# EUROPÄISCHE PATENTSCHRIFT

(11) **EP 2 608 841 B1**
(45) Veröffentlichungstag und Bekanntmachung des Hinweises auf die Patenterteilung: **07.12.2016**
(21) Anmeldenummer: 11754295.1
(22) Anmeldetag: 26.08.2011
(51) Int. Cl.: A61N 1/05, H01B 5/00

(54) **ELEKTRODE FÜR MEDIZINISCHE ANWENDUNGEN, SYSTEM MIT EINER DERARTIGEN ELEKTRODE UND VERFAHREN ZUR HERSTELLUNG EINER DERARTIGEN ELEKTRODE**
ELECTRODE FOR MEDICAL APPLICATIONS, SYSTEM HAVING SUCH AN ELECTRODE, AND METHOD FOR PRODUCING SUCH AN ELECTRODE
ÉLECTRODE POUR UTILISATIONS MÉDICALES, SYSTÈME COMPORTANT UNE ÉLECTRODE ET PROCÉDÉ DE FABRICATION D'UNE ÉLECTRODE

(30) Priorität: 26.08.2010 DE 102010035475
(43) Veröffentlichungstag der Anmeldung: 03.07.2013
(73) Patentinhaber: Acandis GmbH & Co. KG, 76327 Pfinztal (DE)
(72) Erfinder: CATTANEO, Giorgio, 76199 Karlsruhe (DE); STETT, Alfred, 72770 Reutlingen (DE); GHARABAGHI, A., 72070 Tübingen (DE)
(74) Vertreter: Kilchert, Jochen
(86) Internationale Anmeldenummer: PCT/EP2011/004307
(87) Internationale Veröffentlichungsnummer: WO 2012/025245

(56) Entgegenhaltungen:
- WO-A1-2005/110528
- WO-A2-2007/105060
- US-A1- 2007 106 359

## Beschreibung

Die Erfindung bezieht sich auf eine Elektrode für medizinische Anwendungen zur Neuromodulation und/oder Nervenstimulation und/oder neurologischen Signalerfassung, die zur Einfuhr in ein Hohlorgan eines Körpers komprimierbar und expandierbar ist und mit einer Stromversorgung gekoppelt oder koppelbar ist. Eine gattungsgemäße Elektrode ist beispielsweise aus US 5,531,779 bekannt.

Die bekannte Elektrode wird zur Behandlung von Herzfehlern eingesetzt und ist zur Übertragung relativ hoher Ströme ausgelegt, die für derartige Behandlungen erforderlich sind. Die Elektrode ist als intravaskulärer Stent ausgebildet, der aus einer Stahllegierung hergestellt und insgesamt leitfähig ist. Der gesamte Stent bildet damit die Sonde, die zur Einfuhr in den Körper komprimierbar und expandierbar ist. Zur Erzeugung eines elektrischen Feldes wird die Stentelektrode mit einer weiteren Elektrode kombiniert, die beispielsweise in Form einer zweiten Stentelektrode ausgebildet ist, die von der ersten Stentelektrode beabstandet implantiert wird.

Die bekannte Stentelektrode ist aus einzelnen Drähten aufgebaut, die im expandierten Zustand im Wesentlichen parallel verlaufen und korbförmig ausgebildet sind. Andere auf Drähten basierende Stentstrukturen, wie beispielsweise gewebte oder geflochtene Stents werden als geeignet offenbart. Derart gestaltete Stents werden in der Praxis mit Hilfe von Flechtmaschinen hergestellt, wobei mehrere Drähte zu einem zylinderförmigen Geflecht zusammengeführt werden.

Aus der Praxis sind im Allgemeinen Stents bekannt, die aus einem rohrförmigen Vollmaterial durch ein materialabtragendes Verfahren, beispielsweise ein Laserschneidverfahren hergestellt sind. Sowohl die bekannten drahtgeflochtenen, als auch lasergeschnittene Strukturen weisen ungleichmäßige bzw. unregelmäßige elektrische Eigenschaften auf. Für kleine Ströme bzw. Spannungen sind die bekannten Strukturen, insbesondere die Elektrode gemäß US 5,531,779, daher schlecht geeignet. Die Herstellung mehrerer Elektroden mit gleichen bzw. gleichartigen elektrischen Eigenschaften ist erschwert, da die eingesetzten Materialien lokal unterschiedliche Leitfähigkeiten aufweisen.

Aus US 2003/74039 A1 ist eine Elektrode bekannt, die zur Herzbehandlung eingesetzt wird. Die Elektrode wird mit einer weiteren zusätzlichen Elektrode verwendet, um ein elektrisches Feld zu erzeugen.

Ferner sind Elektroden bekannt, bei denen die Elektrodenpole in ein und denselben implantierbaren Körper integriert sind, sodass die zweite separate Elektrode entfallen kann. US 6,445,953 B1 offenbart beispielsweise eine Stentelektrode, an deren Außenfläche eine erste und zweite Elektrode befestigt sind, die durch ein Funksignal angesteuert werden. Ein weiteres Beispiel für eine implantierbare Elektrode, bei der die beiden Pole im selben Implantat angeordnet sind, ist aus WO 2008/094344 A1 bekannt, die einen spiralförmigen Draht offenbart, an welchem verschiedene Elektrodenbereiche bzw. Pole vorgesehen sind. Die verschiedenen Elektrodenbereiche können unabhängig voneinander angesteuert werde, sodass das mit der Elektrode erzeugbare elektrische Feld modulierbar ist.

Eine ähnliche Elektrode ist aus WO2008/094789 A1 bekannt, die zur Verankerung des Elektrodendrahtes eine Stentstruktur aufweist. Diese ist mit dem Elektrodendraht verbunden, sodass durch die Expansion des Trägerstents der Elektrodendraht an die Gefäßwand gepresst wird. Der Stent dient dabei ausschließlich als mechanische Stütze.

Eine weitere Elektrode aus einem Draht mit unterschiedlichen polarisierbaren Elektrodenbereichen ist aus EP 1 304 135 A2 bekannt. Außerdem beschreiben US 2007/106359 A1 und WO 2005/110528 A1 stentartige Strukturen, die als Elektroden wirken.

Die Herstellung der vorstehend genannten Elektroden ist kompliziert, da hierfür entweder aufwändige Fügetechniken notwendig sind oder die Stromversorgung der einzelnen Elektrodenbereiche schwierig zu realisieren ist. Außerdem sind die mit der Stützstruktur verbundenen Elektroden verhältnismäßig groß, sodass einerseits die Anzahl der Elektroden limitiert ist und andererseits eine präzise Einstellung der elektrischen Felder nur eingeschränkt möglich ist. Außerdem sind für einige der bekannten Systeme Leitungen erforderlich, die mit der Elektrode verbunden sind und entsprechend isoliert sein müssen, da andernfalls ein Kontakt mit der tragenden Struktur der Elektrode zu einem Kurzschluss führen würde. Die isolierten Leitungen erhöhen die Komplexität und Sperrigkeit der Systeme und erschweren die Zufuhr, insbesondere in kleine Gefäße. Außerdem kann es zu unregelmäßigen Oberflächen kommen, wodurch die Verletzungsgefahr steigt. Der Nachteil bei konventionellen Elektroden besteht auch in der Schwierigkeit, auf einer Elektrode viele Schaltkreise unterzubringen.

Bei der herkömmlichen schmelztechnischen Herstellung von Drähten für bekannte Elektroden werden unvermeidbar relativ große Einschlüsse im Materialgefüge gebildet. Diese Einschlüsse entstehen in der Schmelze, wachsen bei Erkalten der Schmelze und liegen dann im erstarrten Material vor. Die schmelztechnisch verursachten Einschlüsse können beispielsweise bei Nickel/Titan-Legierungen Titan, Nickel und dazu Kohlenstoff und/oder Sauerstoff beinhalten. Die Größe der Einschlüsse beträgt in der Praxis einige µm, insbesondere 5 µm bis mehr als 20 µm.

Im Rahmen der Anmeldung werden unter Einschlüssen (inclusions) schmelztechnisch gebildete Phasen verstanden, in welchen neben Titan und Nickel Fremdelemente, wie beispielsweise Kohlenstoff und/oder Sauerstoff vorliegen. Diese Fremdelemente können in den Rohrmaterialien vorhanden sein bzw. von außen, beispielsweise durch den Tiegel oder den Schmelzprozess in das Material eingetragen werden. Bei herkömmlich hergestelltem Material kann es zusätzlich zu den Einschlüssen bei der Wärmebehandlung zu Ausscheidungen

(precipitates, precipitations) von Phasen aus der festen Lösung kommen, die durch mehrfache Glühbehandlungen und/oder durch Umformung des Materials wachsen können.

Insgesamt unterscheiden sich Ausscheidungen von Einschlüssen zumindest durch ihre Entstehung. Ausscheidungen bilden sich als Phase aus der festen Lösung, also aus dem kristallinen Material. Mit anderen Worten werden Ausscheidungen aus dem kristallinen Material ausgeschieden. Einschlüsse hingegen bilden sich in der Schmelze durch Keimwachstum mit Fremdatomen und bleiben im erstarrten Material erhalten bzw. eingeschlossen.

Zusammengefasst werden bei konventionell hergestellten Elektroden aufgrund der Vielzahl der aufeinanderfolgenden Prozesse (Schmelzen und Erstarren, mehrere Warmumformungen und Kaltumformungen mit zwischengeschalteten Wärmebehandlungen, insbesondere Zwischenglühungen) undefiniert viele Gitterfehler induziert, die nicht kontrollierbar sind. Diese Gitterfehler stehen in Wechselwirkung untereinander. Beispielsweise beeinflusst die Dichte der Leerstellen die Versetzungsbildung und die Versetzungsbewegung. Die Kaltverformung bedingt deutlich die Versetzungsbildung und beeinflusst zusammen mit den Glühbehandlungen zusätzlich stark die Korngröße. Damit lässt sich an einem konventionell hergestellten Bauteil weder der elektrische Widerstand genau vorhersagen bzw. einstellen, noch lassen sich Chargenunterschiede bzw. Los-zu-Los-Unterschiede vermeiden.

Der Erfindung liegt die Aufgabe zugrunde, eine Elektrode für intravaskuläre medizinische Anwendungen anzugeben, die verbesserte elektrische Eigenschaften aufweist. Ferner soll mit der Erfindung ein System mit einer derartigen Elektrode und ein Verfahren zur Herstellung einer derartigen Elektrode angegeben werden.

Erfindungsgemäß wird diese Aufgabe im Hinblick auf die Elektrode durch den Gegenstand des Patentanspruchs im Hinblick auf das System durch den Gegenstand des Patentanspruchs 14 und im Hinblick auf das Verfahren durch den Gegenstand des Patentanspruchs 15 gelöst.

Die Erfindung beruht auf dem Gedanken, eine Elektrode für medizinische Anwendungen zur Neuromodulation und/oder Nervenstimulation und oder neurologischen Signalerfassung anzugeben. Die Elektrode ist zur Einfuhr in ein Hohlorgan eines Körpers komprimierbar und expandierbar. Die Elektrode ist ferner mit einer Stromzufuhr gekoppelt oder koppelbar. Die Elektrode umfasst eine komprimierbare und expandierbare Gitterstruktur, die aus Gitterstegen gebildete Zellen aufweist. Die Gitterstruktur ist mit der Stromzufuhr gekoppelt oder koppelbar. Dabei ist die Gitterstruktur, insbesondere schichtweise, durch physikalische Gasphasenabscheidung erhalten.

Die erfindungsgemäße Elektrode unterscheidet sich von den bekannten Systemen, insbesondere von dem System gemäß US 5,531,779 einerseits dadurch, dass die Gitterstruktur aus Gitterstegen ausgebildet ist. Die Gitterstege bilden bzw. begrenzen Zellen. Somit handelt es sich bei der erfindungsgemäßen Gitterstruktur nicht um eine Gitterstruktur auf der Basis von Einzeldrähten, wie bei einem Geflecht oder Gewebe, sondern um eine Gitterstruktur auf der Basis von Gitterstegen. Die Gitterstege sind dabei vorzugsweise in derselben bzw. in einer einzigen Wandungsebene fluchtend zueinander angeordnet. Ferner sind die Gitterstege im Unterschied zu Einzeldrähten in Kreuzungspunkten miteinander fest verbunden, insbesondere stoffschlüssig verbunden.

Andererseits unterscheidet sich die erfindungsgemäße Elektrode von bekannten Elektroden dadurch, dass die Gitterstruktur, insbesondere schichtweise, durch physikalische Gasphasenabscheidung (PVD) erhalten ist. Die bekannten Elektroden weisen als Gitterstrukturen Geflechte auf, bei denen die Drähte dagegen schmelzmetallurgisch und durch Verformung hergestellt sind. Dasselbe gilt für lasergeschnittene Gitterstrukturen. Daraus ergeben sich feststellbare Unterschiede zwischen dem Gefüge der erfindungsgemäßen Elektrode und dem Gefüge bekannter Elektroden. Bei bekannten Elektroden kommt es beispielsweise durch die Umformschritte bei der Herstellung zu einer Zug- oder Walztextur, die auf Grund der PVD-Herstellung im Gefüge der erfindungsgemäßen Elektrode nicht vorhanden ist. Ferner unterscheidet sich die erfindungsgemäße Elektrode von bekannten Elektroden durch die fehlende Zeiligkeit der Ausscheidungen. Bei bekannten Elektroden sind die Ausscheidungen anisotrop, wobei die Ausscheidungen im wesentlichen in Verformungsrichtung ausgerichtet sind, weil bspw. pseudoelastische Formgedächtnismaterialien gerade gerichtet verarbeitet werden, insbesondere Rohre oder Drähte. Die wird durch eine Wärmebehandlung unter Spannung erreicht. Bei der erfindungsgemäßen Elektrode sind die Ausscheidungen im wesentlichen isotrop. Generell ist das Gefüge der erfindungsgemäßen Elektrode im wesentlichen isotrop oder zumindest isotroper als das Gefüge der bekannten Elektroden, das im wesentlichen anisotrop ist. Weitere Unterschiede sind in der Gleichmäßigkeit und Größe der Ausscheidungen feststellbar.

Ein besonders deutlicher Unterschied besteht in dem Vorhandensein bzw. in der Größe von Einschlüssen. Bei der erfindungsgemäßen Elektrode sind in der Regel im Gefüge keine Einschlüsse vorhanden. Wenn Einschlüsse vorhanden sind, ist die Größe dieser Einschlüsse kleiner als die Größe von Einschlüssen in schmelzmetallurgisch hergestellten Elektroden. Bei konventionellen Elektrodenmaterialien sind Einschlüsse in der Form von Karbiden und/oder intermetallischen Phasen mit Sauerstoff vorhanden. Die erfindungsgemäße Elektrode unterscheidet sich somit als solche von bekannten Elektroden. Durch die plastische Verformung bei der Herstellung herkömmlicher Elektroden wird das Gefüge um die Einschlüsse herum gestört, was sich auf die Leitfähigkeit der Elektrode auswirkt. Da bei der erfindungsgemäßen Elektrode keine oder nur relative kleine Einschlüsse im Material vorhanden sind, wird das Gefüge nicht oder nur relativ wenig gestört, wenn das Material verformt wird. Die wirkt sich positiv auf die Leitfähigkeit aus.

Erfindungsgemäß ist die gesamte Gitterstruktur durch ein PVD-Verfahren hergestellt. Damit ist eine freitragende Gitterstruktur erhältlich. Alternativ kann auf eine Tragstruktur, die nicht durch ein PVD-Verfahren hergestellt ist, insbesondere auf eine herkömmliche Tragstruktur, eine Schicht mit einer anderen Funktion als die Tragschicht durch ein PVD-Verfahren aufgebracht werden. Wenn nachfolgend die Materialeigenschaften der Gitterstruktur näher erläutert werden, beziehen sich die Ausführungen sowohl auf den Fall, dass nur ein Teil bzw. ein Bereich der Gitterstruktur, bspw. die Schicht oder einzelne Gitterelemente, die Materialeigenschaften aufweist, als auch auf den Fall, dass die gesamte Gitterstruktur, bspw. bei einer freitragenden Gitterstruktur, die Materialeigenschaften aufweist.

Target, können die Gefügeeigenschaften genau eingestellt werden. Die physikalische Gasphasenabscheidung ermöglicht überdies eine wiederholbare Herstellung der Gitterstruktur mit gleichen Eigenschaften. Die besonderen Gefügeeigenschaften bzw. Materialeigenschaften der erfindungsgemäßen Elektrode führen zu einer Verbesserung der elektrischen Eigenschaften, insbesondere der Leitfähigkeit, der Gitterstruktur. Dadurch ist die erfindungsgemäße Elektrode nicht ausschließlich, aber in besonderer Weise für neurologische Anwendungen, insbesondere im Bereich der Sensorik, geeignet.

Unter der Leitfähigkeit der Gitterstruktur bzw. der Elektrode wird einerseits die Leitfähigkeit der mit dem Gewebe in Berührung kommenden Oberfläche der Elektrode, insbesondere der Außenfläche verstanden, um elektrische Impulse von der Elektrode ins Gewebe zu übertragen bzw. vom Gewebe elektrische Signale ohne Verluste zu empfangen und über den Elektrodenkörper als mechanische Stütze an die Auswertung weiterzuleiten. Andererseits wird unter der Leitfähigkeit der Gitterstruktur bzw. der Elektrode auch die Leitfähigkeit in der Elektrode verstanden, also die Leitfähigkeit eines Materials, das die Funktion der Stromführung in der Elektrode übernimmt. Die Leitfähigkeit dieses Materials kann höher sein als die Leitfähigkeit des Trägermaterials der Gitterstruktur bspw. höher als die Leitfähigkeit von Materialien auf der Basis von NiTi.

Durch die Herstellung der Elektrode im PVD-Verfahren wird erreicht, dass sich über die gesamte Gitterstruktur relativ konstante bzw. gleichmäßige elektrische Eigenschaften einstellen, wobei überdies die Eigenschaften bei der Herstellung der Gitterstruktur gezielt kontrolliert werden können. Die erfindungsgemäße Elektrode ermöglicht daher die Erzeugung eines im Wesentlichen homogenen elektrischen Feldes. Durch das PVD-Verfahren ist es auch möglich, die elektrischen Eigenschaften der Elektrode entlang der Gitterstruktur zu variieren, was bei herkömmlichen Elektroden nicht möglich ist. Gleichzeitig wird durch die durch die durch physikalische Gasphasenabscheidung erzielbaren Gefügeeigenschaften eine gute Stabilität der Gitterstruktur erreicht, so dass die Gitterstruktur bzw. allgemein die Elektrode zur Stützung eines Körperhohlorgans geeignet ist. Insbesondere ermöglicht die physikalische Gasphasenabscheidung die Herstellung einer selbsttragenden Elektrode. Die erfindungsgemäße Elektrode weist als sowohl gute mechanische als auch gute elektrische Eigenschaften auf.

Im Unterschied zur schmelztechnischen Herstellung konventioneller Elektroden wird durch das dünnschichttechnologische Verfahren der physikalischen Gasphasenabscheidung ein Material bereitgestellt, das keine oder zumindest eine verringerte Anzahl von Einschlüsse aufweist. Das Material der Gitterstruktur der erfindungsgemäßen Elektrode ist also zumindest bereichsweise, insbesondere im gesamten Bereich der Gitterstruktur im Wesentlichen frei von Einschlüssen, da das Material nicht unmittelbar aus einer Schmelze, sondern durch Abscheidung eines zerstäubten Targetmaterials auf einem Substrat hergestellt ist. Die bereichsweise Einstellung der Materialeigenschaften wird dadurch erreicht, dass ein oder mehrere Bereiche durch ein PVD-Verfahren hergestellt werden. Es wird davon ausgegangen, dass durch die physikalische Gasphasenabscheidung die erzielbaren Gehalte an Sauerstoff und Kohlenstoff niedriger als bei herkömmlich auf schmelztechnischem Wege hergestellten Materialien ist, da die physikalische Gasphasenabscheidung im Vakuum erfolgt.

Falls es bei der Herstellung des Materials der Gitterstruktur mit dem physikalischen Gasphasenabscheideverfahrens dennoch zu Einschlüssen durch Verunreinigungen kommen sollte, sind diese bei der Gitterstruktur der erfindungsgemäße Elektrode wesentlich kleiner als bei Gitterstrukturen, die durch herkömmliche Schmelzverfahren hergestellt sind. Die Einschlüsse im Material der Gitterstruktur betragen höchstens 1µm, insbesondere höchstens 500 nm, insbesondere höchstens 200 nm, insbesondere höchstens 100 nm, insbesondere 75 nm, insbesondere 50 nm, insbesondere 25 nm.

Grundsätzlich lässt sich durch das Vorhandensein von Einschlüssen, der Gleichmäßigkeit, Größe und Zeiligkeit von Ausscheidungen, sowie dem Vorhandensein einer Zug-bzw. Walztextur erkennen, ob eine Elektrode oder Gitterstruktur konventionell oder mittels Gasphasenabscheidung hergestellt wurde.

Ausscheidungen im Material der Gitterstruktur der erfindungsgemäßen Elektrode bestehen aus den Legierungskomponenten und enthalten üblicherweise keine von außen eingetragenen Fremdatome. Beispielsweise bestehen Ausscheidungen bei Materialien aus Nickel-Titan-Legierungen in der Regel aus Titan und Nickel, wobei es verschiedene Ausscheidungstypen mit verschiedenen Zusammensetzungen gibt. Typische mit der physikalischen Gasphasenabscheidung erhältliche Ausscheidungen umfassen Ni₄Ti₃.

Ein weiterer Unterschied, der die unterschiedlichen Herstellungsverfahren am Material erkennbar macht, besteht darin, dass die Einschlüsse und Ausscheidungen bei konventionell schmelztechnisch bzw. konventionell verarbeiteten Materialien zeilenförmig in Richtung der Zugrichtung (Rohr oder Draht) bzw. der Walzrichtung (Blech) angeordnet sind. Hingegen sind die Ausscheidungen des erfindungsgemäßen Materials der Gitterstruktur isotrop ausgerichtet. Die isotrope, also ungerichtete Ausrichtung der Ausscheidungen wirkt sich positiv auf die elektrischen Eigenschaften, insbesondere die Leitfähigkeit und/oder die Homogenität der Leitfähigkeit und/oder die Einstellbarkeit Leitfähigkeit der Elektrode aus. Darüberhinaus enthalten alle konventionell hergestellten Strukturen Umformtexturen, dh Walz- oder Zugtexturen mit einer Vorzugsrichtung der Kristallite.

Gegenüber den bekannten Elektroden, die auf schmelztechnischem Wege hergestellt sind, weist die erfindungsgemäße Elektrode, die durch ein physikalisches Gasphasenabscheideverfahren hergestellt ist, bereichsweise weniger Kohlenstoffbestandteile und weniger Sauerstoffbestandteile als konventionelles Material auf. Gitterbaufehler lassen sich bei dem durch physikalische Gasphasenabscheidung hergestellten Material besser kontrollieren. Die mit der konventionellen Herstellung verbundenen Warmumformschritte, bis zu 50 und mehr Kaltumformungen und Zwischenglühprozesse und die daraus resultierenden, nicht kontrollierbaren Gitterfehlerzustände werden vermieden. Dasselbe gilt für die Größe von Korngrenzen innerhalb des Materials, die sich ebenfalls durch das erfindungsgemäße vorgesehene Herstellungsverfahren besser kontrollieren lässt. Die Homogenität aller, die elektrischen Eigenschaften beeinflussenden Gitterbaufehler ist wesentlich besser als bei konventionell hergestelltem Material. Die Grundlage dafür ist, dass nach dem Erschmelzen des Ausgangsmaterials (bspw. NiTi) nur noch die Physikalische Gasphasenabscheidung und (gegebenenfalls) eine einzige Glühbehandlung erfolgt. Die Schwankungsbreite der Dichte von Leerstellen, Versetzungen oder Korngrenzen über den Materialquerschnitt ist gering. Einschlüsse werden vermieden.

Vorzugsweise ist vorgesehen, dass das Material der Gitterstruktur zumindest bereichsweise eine durch physikalische Gasphasenabscheidung gezielt eingestellte maximale Dichte an Materialinhomogenitäten, wie Gitterbaufehler, aufweist. Das Material der Gitterstruktur kann auch, insbesondere zusätzlich, eine gezielt eingestellte maximale Dichte an Grenzflächenfehlern und/oder Volumendefekten aufweisen. Dadurch unterscheidet sich das Material der Gitterstruktur weiter von herkömmlich aus Vollmaterial, beispielsweise durch Umformen, hergestellten Elektroden, die eine größere Dichte an Materialinhomogenitäten als durch physikalische Gasphasenabscheidung hergestelltes Material aufweisen. Fehler im Gefüge des Werkstoffes, also Materialinhomogenitäten, wie Gitterbaufehler (beispielsweise Leerstellen, Zwischengitteratome, Stufenversetzung, Schraubenversetzung, Stapelfehler) und/oder Grenzflächenfehler und/oder Volumendefekte, behindern den Ladungstransport und verringern die Leitfähigkeit. Somit wird durch die aufgrund der physikalischen Gasphasenabscheidung verringerte Dichte an Materialinhomogenitäten eine insbesondere für die neurologische Anwendung bzw. Sensorik vorteilhafte gleichmäßige Leitfähigkeit der Gitterstruktur bzw. allgemein der Elektrode erreicht. Das Material der komprimierbaren und expandierbaren Gitterstruktur ist durch physikalische Gasphasenabscheidung hergestellt derart, dass das Material im Wesentlichen frei von Verformungstexturen und/oder frei von einer Zeiligkeit der Ausscheidungen und/oder frei von Einschlüssen ist.

Bei einer bevorzugten Ausführungsform der erfindungsgemäßen Elektrode beträgt die mittlere Korngröße des Materials der Gitterstruktur zumindest bereichsweise weniger als 4 µm, insbesondere weniger als 3 µm, besonders bevorzugt weniger als 2 µm, insbesondere weniger als 1,5 µm, insbesondere weniger als 1 µm, insbesondere weniger als 0,5 µm, insbesondere weniger als 250 nm. Diese Korngrößen sind im Unterschied zu konventionellen Elektrodenmaterialien in einem Gefüge ohne Verformungstexturen erzielbar. Mittlere Korngrößen herkömmlich hergestellten Materials für Elektroden betragen etwa 5 µm bis 20 µm. Dabei handelt es sich um Großwinkelkorngrenzen im ehemaliger Austenitkörner.

Bei erfindungsgemäßem Elektrodenmaterial kommt es zu geringeren Schwankungen in der Korngröße als bei konventionellem, schmelztechnisch hergestelltem Material. Dies gilt sowohl innerhalb eines Bauteils, als auch von Los-zu-Los bzw. Charge-zu-Charge sowie innerhalb eines Loses. Die Korngröße ist homogen, insbesondere homogener als bei schmelztechnisch hergestelltem Material. Durch die homogene mittlere Korngröße wird eine gleichmäßige und kontrollierbare Leitfähigkeit des Materials der Gitterstruktur erreicht.

Zur Beeinflussung bzw. Einstellung der Größe der Ausscheidungen und/oder der Dichte an Inhomogenitäten, insbesondere linienförmigen Gitterbaufehlern und/oder Grenzflächenfehlern und/oder Volumendefekten, ist vorteilhaft vorgesehen, die Gitterstruktur zumindest teilweise durch Sputtern oder durch lasergestützte Abscheidung herzustellen. Durch die Variation einzelner Prozessparameter des Gasphasenabscheidungsverfahrens können die Inhomogenitäten im Material der Elektrode gezielt eingestellt werden. Dafür geeignete Prozessvariablen sind beispielsweise der Kammerdruck in der Prozesskammer und/oder der Abscheidedruck und/oder der Partialdruck des Prozessgases und/oder die Targetzusammensetzung.

Durch Variation derartiger Parameter sind in an sich bekannter Weise die Größe der Ausscheidungen bzw. Gitterbaufehler sowie deren Dichte gezielt einstellbar. Ferner kann die Korngröße gezielt eingestellt werden. Durch elektronenmikroskopische Prüfverfahren, insbesondere unter Verwendung eines Rasterelektronenmikroskops bzw. eines Transmissionselektronenmikroskops, können die gezielt eingestellten Inhomogenitäten bzw. Gitterbaufehler, generell die Gefügeeigenschaften kontrolliert werden. Die Qualität und Quantität der gemessenen bzw. ermittelten Gitterbaufehler kann zur Einstellung der Prozessparameter des Gasphasenabscheideverfahrens eingesetzt werden.

Das Metall der Gitterstruktur umfasst vorzugsweise eine Formgedächntislegierung auf der Basis NiTi (X) oder Stahl oder eine Chrom-Kobalt-Legierung oder ein biologisch abbaubares Material. Kombinationen der vorgenannten Materialien sind ebenfalls möglich.

Die Verwendung von Formgedächtnismaterialien hat sich insbesondere in der Medizintechnik als vorteilhaft erwiesen. Die Herstellung der Komponenten, in diesem Fall der Gitterstruktur, aus einem Formgedächtnismaterial, erfolgt durch die physikalische Gasphasenabscheidung. Gegenüber herkömmlich hergestellten Formgedächtniskomponenten weist die durch Gasphasenabscheidung hergestellte Gitterstruktur aus einem Formgedächtnismaterial Vorteile auf. Bei herkömmlichen Formgedächtniskomponenten erfolgt die Herstellung durch Vakuumschmelzen, Warmumformung, Kaltumformung, ggf. Laserschneiden, Erodieren oder andere Verfahren der Bearbeitung und abschließende Formgebung. Dabei treten im Rahmen von Gefügeumwandlungen Volumendefekte, beispielweise Gitterleerstellen und große Einschlüsse auf, die verfahrenstechnisch nicht kontrollierbar sind.

Generell erfüllt das Material der Gitterstruktur der Elektrode eine Doppelfunktion. Einerseits beeinflusst das Material der Gitterstruktur die mechanischen Eigenschaften, insbesondere die Stabilität und Flexibilität der Elektrode. Andererseits ist das Material der Gitterstruktur maßgeblich an der Qualität der elektrischen Eigenschaften, insbesondere der Leitfähigkeit der Elektrode beteiligt. Die Doppelfunktion des Materials der Gitterstruktur wird durch die Verwendung eines Formgedächtnismaterials sowohl bezüglich der mechanischen Eigenschaften als auch bezüglich der elektrischen Eigenschaften verbessert. Insbesondere weist die Gitterstruktur aufgrund des pseudoelastischen Effekts des Formgedächtniswerkstoffs gute mechanische Eigenschaften, gerade hinsichtlich der Flexibilität, auf. Die Gitterstruktur ist in der Regel selbstexpandierbar. Prinzipiell kann die Aufweitung durch den temperaturgesteuerten Formgedächtniseffekt hervorgerufen werden. Mechanisch expandierbare, insbesondere ballonexpandierbare Gitterstrukturen sind möglich.

Bei der Elektrode kann mit einer Verbesserung des Ermüdungsverhaltens der Gitterstruktur gerechnet werden, was insbesondere hinsichtlich der Expansionsfunktion der Gitterstruktur zweckmäßig ist. Als besonders vorteilhaft hat sich ein Formgedächtniswerkstoff erwiesen, der eine Nickel-Titan-Legierung umfasst, wobei die Nickel-Titan-Legierung vorteilhafterweise eine Zusammensetzung aus 50,8 Atom-% Nickel und 49,2 Atom-% Titan aufweist.

Die Gitterstruktur kann zusätzlich oder alternativ zu einem Formgedächtnismaterial ein biologisch abbaubares Material umfassen. Geeignete biologisch abbaubare Materialien können beispielsweise Magnesium und/oder Kalzium, und/oder Zink aufweisen. Auch bioabbaubare Polymere sind denkbar. Mit einem biologisch abbaubaren Material ist eine Elektrode herstellbar, die sich zum temporären Einsatz in einem Körperhohlorgan eignet. Konkret kann die Elektrode in ein Körperhohlorgan eingebracht und zur Abgabe oder Erfassung elektrischer Signale genutzt werden, wobei die Funktion der Elektrode zeitlich begrenzt ist. Die Elektrode wird biologisch abgebaut, so dass eine zeitlich begrenzte Behandlung ermöglich ist, wobei ein erneuter operativer Eingriff zur Entfernung der Elektrode vermieden wird. Zusätzliche bzw. weitere operationsbedingte gesundheitliche Risiken werden somit vermieden. Es ist auch möglich nur den Träger aus biologisch abbaubarem Material herzustellen, so dass nach dem Einwachsen der Elektrode, bzw. des elektrisch aktivierbarem Teils der Elektrode der auflösbare Träger ganz verschwunden ist.

Die Gitterstruktur kann zumindest in einem Herstellzustand rotationssymmetrisch, insbesondere hohlzylindrisch, ausgebildet sein. Als Herstellzustand wird im Rahmen der Anmeldung der Zustand der Gitterstruktur bezeichnet, in dem im Wesentlichen keine äußeren Kräfte auf die Gitterstruktur wirken, die eine Komprimierung herbeiführen können. Mit anderen Worten entspricht der Herstellzustand einem kraftunbelasteten Zustand der Gitterstruktur. Die Gitterstruktur liegt im Herstellzustand vollständig expandiert vor. Im Allgemeinen kann die Gitterstruktur stentartig ausgebildet sein. Die Elektrode kann eine Stentelektrode sein. Durch die rotationssymmetrische bzw. hohlzylindrische Form bietet die Gitterstruktur gute Voraussetzungen für die Implantation bzw. den temporären Einsatz in Körperhohlorganen, insbesondere Blutgefäßen.

Gemäß einem weiteren bevorzugten Ausführungsbeispiel ist vorgesehen, dass die Gitterstruktur mehrere, insbesondere wenigstens zwei, in radialer Richtung aufeinander angeordnete Schichten, insbesondere mit unterschiedlichen Funktionen, aufweist. Die unterschiedlichen Funktionen der Schichten können beispielsweise durch unterschiedliche Werkstoffe und/oder durch unterschiedliche Gefügeeigenschaften zur Bildung der Schichten bedingt sein. Mit anderen Worten kann die Gitterstruktur unterschiedliche Schichten aufweisen, die jeweils ein voneinander verschiedenes Material aufweisen. Dabei ist andererseits nicht ausgeschlossen, dass die Schichten dasselbe Material umfassen. Die unterschiedlichen Schichten sind vorzugsweise in radialer Richtung aufeinander angeordnet. Die Gitterstruktur kann also wenigstens eine Außenschicht aufweisen, die sich über einen Außenumfang der Gitterstruktur erstreckt. Zusätzlich kann eine Innenschicht vorgesehen sein, die sich über einen Innenumfang der Gitterstruktur erstreckt. Die beiden Schichten, also die Außenschicht und die Innenschicht, sind somit konzentrisch bzw. koaxial ineinander angeordnet. In radialer Richtung betrachtet liegen die beiden bzw. mehreren Schichten aufeinander bzw. sind aufeinander angeordnet.

Die Gitterstruktur kann insbesondere wenigstens eine elektrisch leitende Schicht und wenigstens eine mechanisch tragende Schicht, insbesondere aus unterschiedlichen Materialien, aufweisen. Die zuvor erläuterte Doppelfunktion der Gitterstruktur bzw. des Materials der Gitterstruktur kann also unterschiedlichen Schichten zugeordnet sein. Dabei ist nicht ausgeschlossen, dass eine der Schichten zusätzlich die Doppelfunktion, nämlich verbesserte mechanische und verbesserte elektrische Eigenschaften umfasst. Bspw. sind Legierungen auf NiTi Basis elektrisch leitend.

In einer besonders bevorzugten Ausgestaltung der Erfindung ist die elektrisch leitende Schicht außen zur Berührung mit dem Hohlorgan angeordnet und die mechanisch tragende Schicht bezogen auf die elektrisch leitende Schicht radial weiter innen angeordnet. Mit anderen Worten kann die Innenschicht als mechanisch tragende Schicht und die Außenschicht als elektrisch leitende Schicht ausgebildet sein. Die Anordnung der elektrisch leitenden Schicht außen bzw. auf einem Außenumfang der Gitterstruktur ist im Hinblick auf eine effiziente elektrische Kontaktierung mit einer Gefäßwand eines Körperhohlorgans besonders vorteilhaft. Im Gebrauch stützt sich die Gitterstruktur vorzugsweise an eine Innenfläche der Gefäßwand eines Körperhohlorgans ab, so dass sich eine gute und stabile elektrische Kontaktierung zwischen der Elektrode und dem Körperhohlraum einstellt.

Die Gitterstruktur kann wenigstens eine röntgensichtbare Schicht aufweisen. Damit wird vorteilhaft erreicht, dass die Positionierung der Elektrode bzw. der Gitterstruktur in einem Körperhohlorgan von einem externen Standpunkt beobachtbar und somit kontrollierbar ist. Durch die verbesserte Röntgensichtbarkeit der Gitterstruktur ist eine genaue Positionierung der Elektrode sichergestellt. Insgesamt verbessert die röntgensichtbare bzw. fluoroskopisch sichtbare Schicht die Handhabbarkeit der Elektrode. Anhand der Metalle Platin und Gold wird deutlich, dass eine röntgensichtbare Schicht gleichzeitig die Leitfähigkeit übernehmen kann.

Die elektrisch leitende Schicht kann ferner mit einer elektrisch isolierenden Schicht, insbesondere einer Isolierschicht, verbunden sein. Die elektrisch isolierende Schicht ist vorzugsweise auf einer radial innen oder einer radial außen angeordneten Seite der elektrisch leitenden Schicht angeordnet. Ferner kann die elektrisch isolierende Schicht zwischen zwei Schichten angeordnet sein, die mit denselben oder unterschiedlichen elektrischen Spannungen beaufschlagbar sind. Die beiden die elektrisch isolierende Schicht umhüllenden Schichten können durch die Außenschicht und die Innenschicht gebildet sein. Durch die elektrisch isolierende Schicht sind die beiden umhüllenden Schichten galvanisch voneinander getrennt. Die durch die elektrisch isolierende Schicht getrennten Schichten können mit unterschiedlichen elektrischen Spannungen beaufschlagt werden. Insbesondere kann eine der beiden Schichten, die durch die elektrisch isolierende Schicht elektrisch getrennt sind, mit einer negativen Spannung und die andere Schicht mit einer positiven Spannung beaufschlagt werden. Eine der beiden elektrischen Spannungen kann Null betragen. Vorteilhafterweise wird die Beaufschlagung der durch die elektrisch isolierende Schicht getrennten Schichten durch die Materialeigenschaften der Schichten eingestellt. Beispielsweise können die beiden durch die elektrisch isolierende Schicht getrennten Schichten unterschiedliche elektrische Widerstände aufweisen.

In allgemeiner Form wird also eine Elektrode offenbart und beansprucht, deren komprimierbare und expandierbare Gitterstruktur aus Gitterstegen schichtweise aufgebaut ist, wobei wenigstens eine Schicht auf der Außenfläche der Gitterstruktur angeordnet ist, die zum Senden von Impulsen und Empfangen von elektrischen Signalen angepasst ist. Die Gitterstruktur weist wenigstens eine weitere Schicht auf, die zur Stromführung angepasst ist und die auf der Außenfläche der Gitterstruktur angeordnete Schicht mit einer Stromversorgung koppelt oder zur Kopplung angepasst ist. Die weitere Schicht kann auf der Außenfläche der Gitterstruktur angeordnet und ebenfalls zum Senden von Impulsen und Empfangen von elektrischen Signalen angepasst sein. Die weitere Schicht kann in der Gitterstruktur verlaufen und somit eine reine oder zumindest überwiegende Versorgungsfunktion ausüben. Es ist auch möglich, dass die weitere Schicht zur Stromversorgung der mechanisch tragenden Schicht entspricht. Falls erforderlich, sind die leitenden Schicht durch Isolierschichten getrennt.

Durch den Einsatz der Dünnschichttechnik im Zusammenhang mit dem PVD-Verfahren ist es somit möglich,
- elektrische Funktionen, wie z.B. das Senden von Impulsen und das Empfangen von elektrischen Signalen auf sehr kleine Bereiche der Elektrode einzustellen, dh sehr viele Sender und/oder Empfänger auf der Elektrode anzubringen
- die Struktur der Gitterstruktur dazu zu nutzen, um eine räumliche Trennung zwischen Sender/Empfänger herbeizuführen, bzw
- lokale Ausrichtungen der Sender und/oder Empfänger-Punkte vorzunehmen
- sogar innerhalb eines Steges der Gitterstruktur Sender und Empfänger anzubringen

Bei einer weiteren bevorzugten Ausführungsform der erfindungsgemäßen Elektrode ist die Gitterstruktur mit wenigstens einem Medikament beladen, das durch Anlegen einer elektrischen Spannung freisetzbar bzw. aktivierbar ist. Somit kann die lokale Abgabe von Medikamenten bzw. therapeutisch wirksamen Substanzen zeitlich gesteuert werden. Dies ermöglicht beispielsweise eine kontinuierlich zeitversetzte Abgabe von Medikamenten, wodurch sich neue therapeutische Möglichkeiten eröffnen. Die zeitgesteuerte Abgabe von Medikamenten kann automatisiert steuerbar sein. Beispielsweise kann ähnlich wie bei bekannten Insulinpumpen, eine Medikamentenabgabe einerseits zeitlich zu bestimmten Tageszeiten erfolgen. Andererseits kann eine Medikamentenabgabe manuell auslösbar sein. Somit bietet diese Ausführungsform der erfindungsgemäßen Elektrode eine verbesserte Möglichkeit für eine bedarfsgerechte Medikation.

Die mechanisch tragende Schicht der Gitterstruktur kann aus einem biologisch abbaubaren Material gebildet sein. Nach dem Einsetzen der Gitterstruktur bzw. allgemein der Elektrode in einem Körperhohlorgan erfolgt üblicherweise eine Endothelialisierung, wobei Körpergewebe die Gitterstege der Gitterstruktur umhüllt. Die Gitterstruktur wächst also in das Gewebe des Körperhohlorgans ein. Ab einem gewissen Endothelialisierungsgrad rückt die tragende bzw. stützende Funktion der mechanisch tragenden Schicht der Gitterstruktur in den Hintergrund, da die Gitterstruktur durch das körpereigene Gewebe des Körperhohlorgans stabilisiert ist. Durch das biologisch abbaubare Material wird die mechanisch tragende Schicht gleichzeitig mit der Endothelialisierung der Gitterstruktur abgebaut. Die elektrisch leitende Schicht bleibt hingegen erhalten und wird durch das körpereigene Gewebe ausreichend stabilisiert. Durch den Abbau der mechanisch tragenden Schicht wird das Gewebe des Körperhohlorgans mechanisch entlastet, so dass, insbesondere bei Blutgefäßen, die ursprüngliche Compliance, also Wandspannung bzw. Elastizität, des Körperhohlorgans zumindest teilweise wiederhergestellt wird. Durch die im Gefäß verbleibende elektrisch leitende Schicht kann die Elektrodenbehandlung bzw. die Signalerfassung fortgesetzt werden.

Gemäß einer weiteren bevorzugten Ausführungsform weisen die Gitterstege der Gitterstruktur eine Außenschicht und eine Innenschicht auf. Die Außenschicht kann eine Verbreiterung umfassen, die sich in Längsrichtung der Gitterstege derart erstreckt, dass die Gitterstege im Bereich der Außenschicht breiter als im Bereich der Innenschicht sind. Durch die Verbreiterung wird die Kontaktfläche zum Körpergewebe erhöht, wodurch die Übertragung elektrischer Signale von der Gitterstruktur in das Gewebe und umgekehrt verbessert wird. Gleichzeitig weist die Gitterstruktur durch die Verbreiterung bzw. durch den vergleichsweise schmaleren Bereich der mechanisch tragenden Schicht eine verbesserte Crimpbarkeit auf. Die Gitterstege bilden durch die Verbreiterung ein im Wesentlichen T-förmiges Querschnittsprofil. Die Kontur der Verbreiterung kann sich entlang des Gittersteges ändern derart, dass die Verbreiterung Ausbauchungen bildet. Die Innenschicht ist dabei schmaler ausgebildet als die Außenschicht. Bei der Komprimierung der Gitterstruktur können die Innenschichten der Gitterstege in Umfangsrichtung aneinander anliegen, wobei sich die Verbreiterung der Gitterstege jeweils überlappt. Insgesamt wird der Querschnittsdurchmesser der Gitterstruktur im komprimierten Zustand somit reduziert.

Vorzugsweise weist die Verbreiterung wenigstens eine elektrisch leitende Schicht und wenigstens eine mechanisch tragende Schicht auf, insbesondere aus unterschiedlichen Materialien. Die elektrisch leitende Schicht kann wie die Außenschicht und die mechanisch tragende Schicht eine Zwischenschicht bilden, die zwischen der Außenschicht und der Innenschicht angeordnet ist.

In einer weiteren bevorzugten Ausgestaltung der erfindungsgemäßen Elektrode ist vorgesehen, dass wenigstens ein Gittersteg, insbesondere mehrere Gitterstege, jeweils mit wenigstens einer Abdeckung verbunden sind, deren Längsrichtung von der Längsrichtung des Gitterstegs abweicht derart, dass sich die Abdeckung in wenigstens eine angrenzende Zelle erstreckt und diese zumindest teilweise abdeckt. Durch die Abdeckung kann die geometrische Form der einzelnen Schichten der Gitterstege variiert werden. Außerdem wird auch bei dieser Ausführungsform die Kontaktfläche zum Hohlorgan hin vergrößert, wodurch eine schonende Behandlung ermöglicht wird, so dass eine Erwärmung des Gewebes begrenzt und Nekrose vermieden wird.

Die Größe und/oder Geometrie der Abdeckungen kann zumindest teilweise gleich oder unterschiedlich sein. Zusätzlich oder alternativ kann die Anzahl der Abdeckungen auf dem Umfang der Gitterstruktur und/oder in Längsrichtung der Gitterstruktur variieren. Damit können die elektrischen Eigenschaften, insbesondere die Modulationseigenschaften, der Elektrode beeinflusst werden.

Die Abdeckungen können elektrisch leitend durch eine Stromzufuhrschicht verbunden, insbesondere miteinander verbunden, sein. Die Stromzufuhrschicht ist an der Außenseite oder der Innenseite der Abdeckungen angeordnet. Die Stromzufuhrschicht kann auf derselben Ebene wie die Abdeckung angeordnet sein. Ferner entspricht die Stromzufuhrschicht der Anordnung der Gitterstege, wobei die an der Innenseite der Abdeckungen angeordnete Stromzufuhrschicht die mechanisch tragende Schicht oder eine von der mechanisch tragenden Schicht elektrisch isolierte Schicht bildet. Die Stromzufuhrschicht kann durch eine elektrisch leitende Schicht gebildet sein, die von der mechanisch tragenden Schicht durch eine Isolierschicht bzw. eine elektrisch isolierte Schicht getrennt ist.

Die außen angeordnete elektrisch leitende Schicht, insbesondere die Verbreiterung oder die Abdeckung, kann zur Vergrößerung der Kontaktfläche strukturiert sein. Beispielsweise kann die außen angeordnete elektrisch leitende Schicht Rillen, Aussparungen, Vorsprünge oder dergleichen Strukturen bis hin zu Nanostrukturen aufweisen, die die Gesamtkontaktfläche zwischen der außen angeordneten elektrisch leitenden Schicht und dem Körpergewebe, insbesondere einer Gefäßwand eines Körperhohlorgans, vergrößern.

Ein mit der Gitterstruktur fest oder lösbar verbundener Führungsdraht kann für die Stromzufuhr angepasst sein. Der konstruktive Aufbau der Elektrode wird somit vereinfacht, da der Führungsdraht einerseits zur Zufuhr der Gitterstruktur an einem Behandlungsort und andererseits als stromzuführendes Element genutzt werden kann.

Ferner kann vorgesehen sein, dass Bereiche der Gitterstruktur nach außen elektrisch isoliert und andere Bereiche der Gitterstruktur nach außen elektrisch leitend ausgebildet sind.

Gemäß einem nebengeordneten Aspekt beruht die Erfindung auf dem Gedanken, ein System mit einer zuvor beschriebenen Elektrode sowie einer Stromversorgung, einem Impulsgeber und einer Steuerelektronik anzugeben, die mit der Elektrode gekoppelt oder koppelbar sind. Sowohl die Elektrode an sich, also unabhängig vom gesamten System, als auch das Gesamtsystem mit einer derartigen Elektrode sind daher Gegenstand der Erfindung.

Das System kann wenigstens eine weitere Elektrode für medizinische Anwendungen umfassen, die mit der erfindungsgemäßen Elektrode bzw. einer Elektrode nach einem der vorstehend genannten erfindungsgemäßen Ausführungsformen zusammenwirkt. Insbesondere ist die weitere Elektrode mit der zuvor beschriebenen Elektrode wirkverbunden oder wirkverbindbar derart, dass eine bipolare Stromführung einstellbar ist. Dadurch werden die Anwendungsmöglichkeiten der Elektrode bzw. allgemein des Gesamtsystems erweitert. Die weitere Elektrode kann beispielsweise eine extrakorporale Elektrode und/oder eine weitere implantierbare Elektrode und/oder die Katheterspitze und/oder der Führungsdraht eines zugehörigen Zuführsystems sein.

Das erfindungsgemäße Verfahren zur Herstellung der zuvor beschriebenen Elektrode beruht darauf, dass die Gitterstruktur zumindest teilweise durch physikalische Gasphasenabscheidung hergestellt wird, wobei ein Material auf einem Substrat schichtweise abgeschieden und derart strukturiert wird, dass eine freitragende Gitterstruktur oder eine beschichtete gitterförmige Tragstruktur gebildet wird. Der Einsatz von physikalischen Gasphasenabscheideverfahren, insbesondere PVD-Verfahren, vorzugsweise Sputterverfahren, zur Herstellung der Elektrode ermöglicht die Ausbildung von Gitterstegen mit verschiedenen Schichten aus unterschiedlichen Materialien. Damit können gezielt Isolierschichten und elektrisch leitende Schichten aufgebracht werden. Das erfindungsgemäßen Verfahren ermöglicht somit eine flexible Herstellung der Elektrode, wobei die unterschiedlichen Schichten je nach Anforderung an die Elektrode angeordnet und im Hinblick auf die geometrische Form der Elektrode ausgebildet werden können. Dabei kann insbesondere eine kombiniertes Ätz-Sputterverfahren zum Einsatz kommen. Außerdem werden besonderes günstige Materialeigenschaften erreicht.

Die Erfindung wird nachfolgend anhand von Ausführungsbeispielen unter Bezugnahme auf die beigefügten, schematischen Zeichnungen, näher erläutert. Darin zeigen:
- Fig. 1: einen Längsschnitt durch die Gitterstruktur einer Elektrode nach einem erfindungsgemäßen Ausführungsbeispiel;
- Fig. 2: eine Draufsicht auf die Elektrode gemäß Fig. 1;
- Fig. 3: einen Längsschnitt durch die Gitterstruktur einer Elektrode nach einem weiteren erfindungsgemäßen Ausführungsbeispiel im implantierten Zustand, wobei die Gitterstege eine Außenschicht und eine Innenschicht aufweisen;
- Fig. 4: einen Längsschnitt durch eine Gitterstruktur einer Elektrode nach einem weiteren erfindungsgemäßen Ausführungsbeispiel im implantierten Zustand, wobei die Gitterstege eine Außenschicht, eine Innenschicht und eine Zwischenschicht umfassen;
- Fig. 5: einen Längsschnitt durch eine Gitterstruktur einer Elektrode nach einem weiteren erfindungsgemäßen Ausführungsbeispiel im implantierten Zustand, wobei die Außenschicht der Gitterstege eine Verbreiterung aufweist;
- Fig. 6: einen Längsschnitt durch eine Gitterstruktur einer Elektrode nach einem erfindungsgemäßen Ausführungsbeispiel im implantierten Zustand, wobei die Verbreiterung mehrere Materialschichten umfasst;
- Fig. 7: einen Längsschnitt durch eine Gitterstruktur einer Elektrode nach einem erfindungsgemäßen Ausführungsbeispiel im implantierten Zustand, wobei die Gitterstege eine Verbreiterung in der elektrisch leitenden Schicht und einen mehrschichtigen Aufbau in der mechanisch tragenden Struktur aufweisen;
- Fig. 8: eine Draufsicht auf eine Elektrode nach einem weiteren erfindungsgemäßen Ausführungsbeispiel, wobei zumindest ein Teil der Gitterstege eine Abdeckung aufweist;
- Fig. 9: eine Draufsicht auf eine Elektrode nach einem weiteren erfindungsgemäßen Ausführungsbeispiel, wobei die Gitterstege unterschiedlich große Abdeckungen umfassen;
- Fig. 10: zwei Ansichten eines Gitterstegs mit Abdeckung;
- Fig. 11: einen Längsschnitt durch die Gitterstruktur einer Elektrode nach einem weiteren erfindungsgemäßen Ausführungsbeispiel, wobei die elektrisch leitende Schicht strukturiert ist;
- Fig. 12: eine Draufsicht auf eine Elektrode nach einem weiteren erfindungsgemäßen Ausführungsbeispiel, wobei unterschiedlich elektrisch leitende bzw. elektrisch isolierende Bereiche vorgesehen sind;
- Fig. 13: eine Draufsicht auf eine Elektrode nach einem weiteren erfindungsgemäßen Ausführungsbeispiel mit Abdeckungen, die gegenüber den freien Gitterstegen eine elektrisch leitende Fläche bereitstellen;
- Fig. 14 a: eine Seitenansicht einer Elektrode nach einem weiteren erfindungsgemäßen Ausführungsbeispiel im implantierten Zustand mit Darstellung des elektrischen Feldverlaufs; und

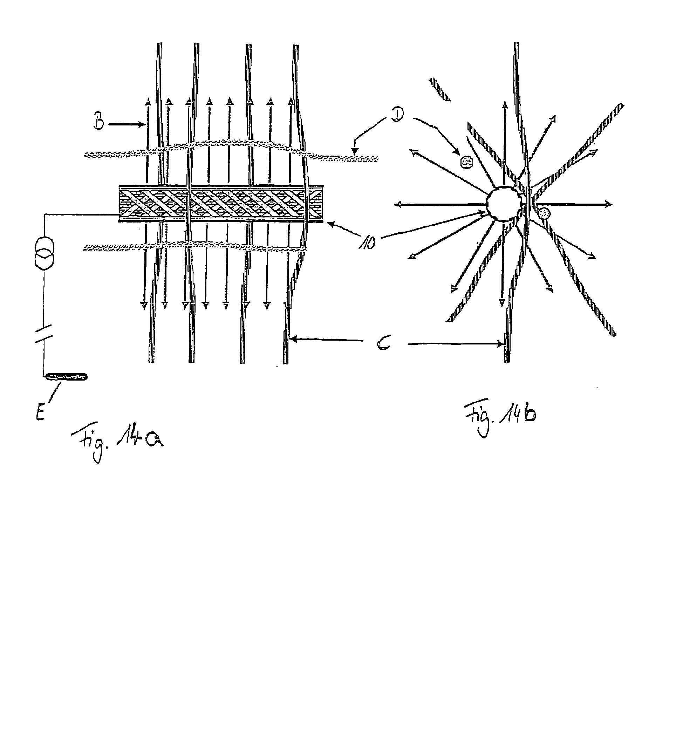

- Fig. 14: b eine Querschnittsansicht durch die Elektrode gemäß Fig. 14 a.

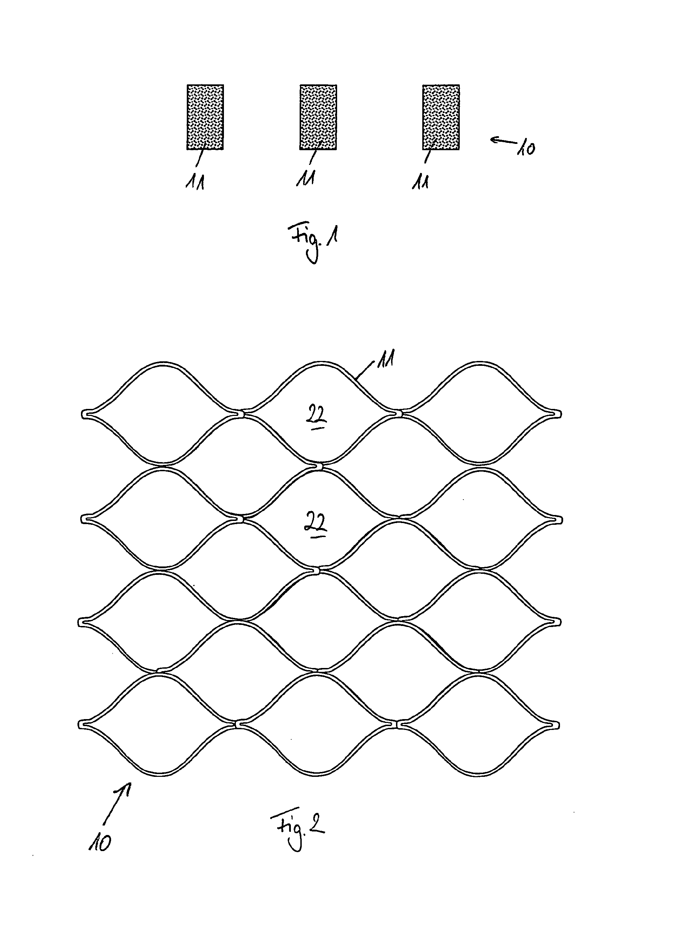

In den Fig. 1 und 2 ist ein Beispiel für eine erfindungsgemäße Elektrode dargestellt, die gleichzeitig verbesserte mechanische Eigenschaften und verbesserte elektrische Eigenschaften aufweist. Die Elektrode ist für medizinische Anwendungen vorgesehen, bei denen die Gitterstruktur in ein Hohlorgan eines Körpers eingeführt wird. Dies können endovaskuläre Anwendungen oder auch andere Anwendungen, wie z.B. in der Harnröhre sein. Die Elektrode, insbesondere deren Dimensionierung, wird an die verschiedenen Anwendungen angepasst.

Die Elektrode ist durch physikalische Gasphasenabscheidung hergestellt.

Die implantierbare Elektrode kann temporär oder permanent in einem Hohlorgan eines Körpers angeordnet werden. Mit anderen Worten ist die Elektrode für eine temporäre oder Permanentanordnung in einem Hohlorgan eines Körpers angepasst.

Die Elektrode weist eine Gitterstruktur 10 auf, die aus Gitterstegen 11 gebildet ist. Die Gitterstege 11 bilden in an sich bekannter Weise Zellen 22. Dazu sind die Gitterstege 11 stellenweise miteinander verbunden bzw. weisen Verbinder auf, die die Stege zur Bildung der Zellen 22 verbinden. Die Gitterstruktur kann eine offene bzw. offen-zellige oder eine geschlossene bzw. geschlossen-zellige Struktur aufweisen. Die unterschiedlichen Strukturen werden als open cell design oder closed cell design unterschieden und sind dem Fachmann bekannt. Die aus Gitterstegen 11 gebildete Gitterstruktur 10 unterscheidet sich von Gitterstrukturen, die als Drahtgeflechte nicht auf der Basis von Gitterstegen 11, sondern auf der Basis von Filamenten oder Drähten gebildet sind. Insbesondere weisen die im Rahmen der Erfindung offenbarten Gitterstrukturen 10 aus Gitterstegen 11 andere mechanische Eigenschaften auf als Drahtgeflechte. Ferner unterscheidet sich die Herstellungsweise von Drahtgeflechten signifikant von der Herstellungsweise von Gitterstrukturen 10, die Gitterstege 11 umfassen. Die erfindungsgemäß erreichbaren Vorteile im Hinblick auf die elektrischen Eigenschaften bzw. die erhöhte Leitfähigkeit der Gitterstruktur 10 sind bei Geflechten nicht erreichbar. Die für die Herstellung von Gitterstrukturen 10 auf der Basis von Gitterstegen 11 eingesetzten Verfahren eignen sich hingegen dazu, eine besonders leitfähige Elektrode auszubilden, die überdies ein vergleichsweise homogenes elektrisches Feld ermöglicht. Zum Einsatz kommt ein Verfahren der physikalischen Gasphasenabscheidung, wodurch die Gitterstruktur 10 sowohl in einer flachen Grundform, als auch in einer dreidimensionalen Grundform, insbesondere einer Zylinderform, schnell herstellbar ist, wobei Gitterbaufehler im Material, insbesondere Ausscheidungen und/oder Einschlüsse, kontrollierbar bzw. einstellbar sind.

Die Gitterstege 11 der Gitterstruktur 10 weisen vorzugsweise ein rechteckiges Querschnittsprofil auf, wie in Fig. 1 dargestellt. Die Kanten der Gitterstege 11 können abgerundet sein. Andere Formen sind möglich. Insbesondere kann das Herstellungsverfahren für die Gitterstruktur 10 einen Ätzschritt umfassen, wobei die Kanten der Gittersteg 11 gezielt geätzt werden. Auf diese Weise können atraumatisch ausgebildete Gitterstege 11 bereitgestellt werden. Die Gitterstege 11 sind ferner elastisch verformbar, so dass die Gitterstruktur 10 von einem komprimierten Zustand in einen expandierten Zustand und umgekehrt überführbar ist. Vorzugsweise entspricht die Form der Gitterstruktur 10 der Form eines Stents. Mit anderen Worten kann die Elektrode eine Stentstruktur aufweisen. Die Gitterstruktur 10 ist vorzugsweise rotationssymmetrisch, insbesondere hohlzylindrisch. Bei dem Ausführungsbeispiel gemäß Fig. 1 und 2 handelt es sich um eine Stentelektrode, d.h. um eine Elektrode mit einer röhrchenförmigen Gitterstruktur 10. Die Erfindung umfasst auch Gitterstrukturen 10 mit einer anderen Form, beispielsweise Filter. Generell kann die Gitterstruktur 10 eine beliebige, kombinierbare und expandierbare Form aufweisen, die dazu geeignet ist, durch ein Zuführsystem in ein Hohlorgan eingebracht zu werden.

Die Gitterstruktur 10 kann auf einem flachen Substrat durch die physikalische Gasphasenabscheidung abgeschieden werden. Alternativ kann das Substrat einen zylinderförmigen Dorn bilden, so dass das Material der Gitterstruktur 10 röhrchenförmig auf das Substrat abgeschieden wird. Bei Verwendung eines flachen Substrats ist es vorteilhaft, die abgeschiedene bzw. fertiggestellte flache Gitterstruktur 10 anschließend in eine Rohrform zu überführen. Dabei können die freien Kanten der Gitterstruktur 10, die sich nach dem Überführen in die Rohrform berühren, miteinander verbunden werden. Die Verbindung kann beispielsweise durch Schweißen, Kleben, Löten und/oder einen Formschluss erfolgen. Ferner kann vorgesehen sein, die Gitterstruktur 10 in eine C-förmige, dreidimensionale Form zu überführen, so dass zwischen den freien Kanten der Gitterstruktur 10 ein Spalt gebildet ist. Die freien Kanten der Gitterstruktur 10 können sich auch überlappen. Mit anderen Worten, kann die Gitterstruktur 10 nach der Herstellung auf einem flachen Substrat um eine Längsachse gerollt werden, wobei sich Bereiche der Gitterstruktur 10 überlappen.

Die Gitterstruktur 10 kann im Allgemeinen selbstexpandierbar ausgebildet sein. Das bedeutet, dass die Gitterstruktur 10 selbsttätig den expandierten Zustand einnimmt, wenn ein äußerer Zwang, der beispielsweise durch das Zuführsystem auf die Elektrode wirkt, entfernt ist. Alternativ kann vorgesehen sein, die Gitterstruktur 10 ballonexpandierbar auszubilden. Dabei ist zur Aufweitung bzw. Expansion der Gitterstruktur 10 eine Kraft erforderlich. Die erforderliche Kraft wird vorzugsweise durch einen aufweitbaren Ballon an der Spitze eines Zuführsystems ausgeübt. Die Gitterstruktur 10 umgibt den Ballon, so dass bei einer Aufweitung des Ballons die Gitterstruktur 10 auseinandergedrückt bzw. in den expandierten Zustand überführt wird.

Die Gitterstruktur 10 der Elektrode kann mehrere Materialschichten mit unterschiedlichen Eigenschaften bzw. Funktionen aufweisen. Die Schichten können aus unterschiedlichen Materialien oder aus denselben Materialien, also denselben chemischen Zusammensetzungen bestehen und in Ihren Gefügeeigenschaften abweichen. Für alle Ausführungsbeispiele und -formen gilt, dass wenigstens eine Schicht zumindest bereichsweise die durch das PVD-Verfahren erreichbaren, in dieser Anmeldung offenbarten Gefügeeigenschaften aufweist. Es ist möglich, dass alle Schichten die gewünschten Gefügeeigenschaften aufweisen. Die Gefügeeigenschaften werden durch das PVD-Verfahren, insbesondere durch Sputtern, eingestellt. Bspw. kann der leitende Bereich, insbesondere die Außenschicht die gewünschten Gefügeeeigenschaften aufweisen und ist durch das PVD-Verfahren hergestellt. Die Tragstruktur ist auf herkömmliche Weise, bspw. durch Laserschneiden, hergestellt. Dies hat den Vorteil, dass die Außenschicht mit den verbesserten Materialeigenschaften mit dem Gewebe in Kontakt kommt. Wenn die gesamte Gitterstruktur in radialer Richtung, also alle übereinander angeordneten Schichten, durch das PVD-Verfahren hergestellt sind, werden sowohl die mechanischen als auch elektrischen Eigenschaften der Elektrode verbessert. Die Schichtdicke mit dem gesputterten Material beträgt mindestens 2%, insbesondere mindestens 5%, insbesondere mindestens 10%, insbesondere mindestens 15%, insbesondere mindestens 20%, insbesondere mindestens 25%, insbesondere mindestens 30%, insbesondere mindestens 35%, insbesondere mindestens 40%, insbesondere mindestens 45%, insbesondere mindestens 50%, insbesondere mindestens 55%, insbesondere mindestens 60%, insbesondere mindestens 65%, insbesondere mindestens 70%, insbesondere mindestens 75%, insbesondere mindestens 80%, insbesondere mindestens 85%, insbesondere mindestens 90%, insbesondere mindestens 95%, insbesondere 100% der Wandstärke der Gitterstruktur.

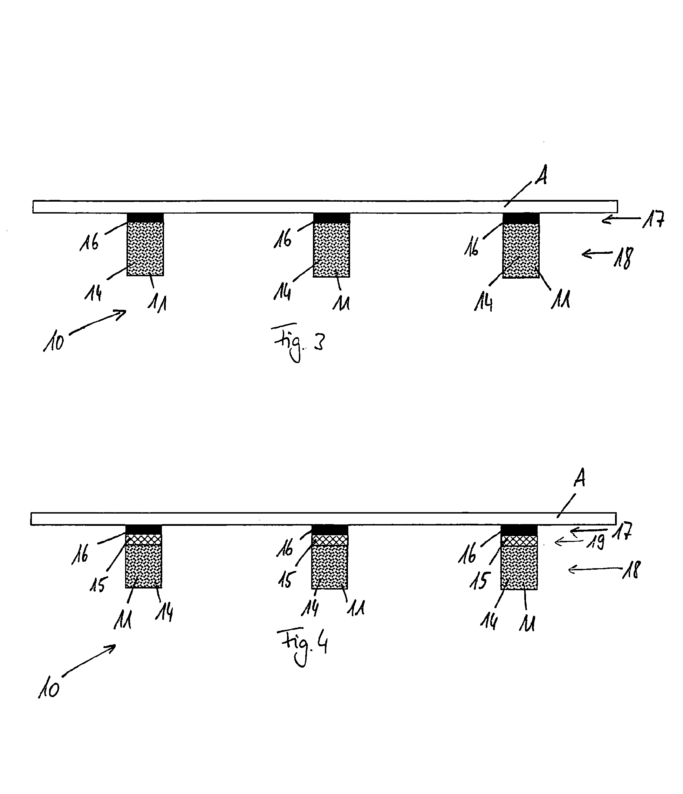

In Fig. 3 ist die Gitterstruktur 10 einer Elektrode im implantierten Zustand dargestellt, die eine Außenschicht 17 und eine Innenschicht 18 umfasst. Die Außenschicht 17 liegt im implantierten Zustand an einem Hohlorgan A an. Vorzugsweise weist die Außenschicht 17 hauptsächlich elektrisch leitende Eigenschaften bzw. eine elektrisch leitende Funktion auf. Die Innenschicht ist hingegen als mechanisch tragende Schicht ausgebildet, d.h. die Innenschicht 18 weist vorwiegend mechanisch tragende Funktionen auf. Zur Verbesserung der elektrisch leitenden Eigenschaften weist die Außenschicht vorzugsweise ein Material auf, das einen verbesserten elektrischen Kontakt zwischen der Elektrode und dem Hohlorgan A erreicht. Ein derartiges Material kann beispielsweise Gold sein. Weitere mögliche Materialien umfassen Silber, Iridium, Iridium-Oxid, Tantal, Titan, Titannitrid, Niob und deren Legierungen, die biokompatibel sind. Die Verwendung von Gold oder Platin oder Tantal für die Außenschicht 17 ist ferner vorteilhaft, da auf diese Weise die Röntgensichtbarkeit der Elektrode bzw. der Gitterstruktur 10 erhöht ist. Die Innenschicht 18 umfasst hingegen vorzugsweise ein Formgedächtnismaterial, insbesondere eine Formgedächtnislegierung, wie beispielsweise Nitinol. Durch die pseudoelastischen Eigenschaften des Formgedächtnismaterials, das im Allgemeinen eine Nickel-Titan-Legierung umfassen kann, wird die Stabilität, insbesondere jedoch die Flexibilität, der Gitterstruktur 10 erhöht. Die Gitterstruktur 10 weist eine gute Verformbarkeit auf, so dass sich die Gitterstruktur 10 an die Struktur des Hohlorgans A anpasst. Die Gitterstruktur 10 stützt sich am Hohlorgan A ab. Auf diese Weise trägt die Innenschicht 18 bzw. allgemein die mechanisch tragende Schicht vorteilhaft dazu bei, den elektrischen Kontakt zwischen der Außenschicht 17 und dem Hohlorgan A zu verbessern. Die mechanisch tragende Schicht kann alternativ oder zusätzlich weitere Materialien, insbesondere Kobalt-Chrom oder Stahl oder ein biodegradierbares Material, umfassen. Die tragende Schicht kann aus einem nicht-leitfähigem Material gebildet sein.

Die Erfindung ist nicht auf zwei Schichten in der Gitterstruktur 10 eingeschränkt. Vielmehr ist es im Rahmen der Erfindung möglich, mehr als zwei Schichten, insbesondere drei, vier, fünf, sechs, sieben, acht oder mehr als acht Schichten für die Bildung der Gitterstruktur 10 vorzusehen. Beispielsweise kann in Betracht gezogen werden, einen mehrschichtigen Aufbau der Gitterstruktur 10 bereitzustellen, wobei die mechanische tragende Schicht zwischen zwei weiteren Schichten angeordnet ist. Die mechanisch tragende Schicht kann also eine Zwischenschicht 19 bilden. Die Zwischenschicht 19 ist vorzugsweise zwischen der Außenschicht 17 und der Innenschicht 18 angeordnet. Wenn die Zwischenschicht 19 durch die mechanisch tragende Schicht gebildet ist, kann vorteilhaft vorgesehen sein, dass die Außenschicht eine elektrisch gut leitende Schicht, beispielsweise umfassend Gold, aufweist, wogegen die Innenschicht 18 eine röntgensichtbare Schicht, beispielsweise aus Tantal oder Platin, umfasst. Alternativ kann die röntgensichtbare Schicht die Zwischenschicht 19 bilden. Die mechanisch tragende Schicht bildet in diesem Fall vorzugsweise die Innenschicht 18. Die Außenschicht 17 umfasst ein elektrisch gut leitendes Material.

Ferner kann vorgesehen sein, dass bei einer Gitterstruktur 10, die wenigstens drei unterschiedliche Schichten aufweist, die Zwischenschicht 19 oder zumindest eine der Zwischenschichten 19 eine Isolierschicht 15 bildet. Die Isolierschicht 15 kann beispielsweise ein Oxid umfassen. In Fig. 4 ist ein Beispiel der Gitterstruktur einer Elektrode im implantierten Zustand dargestellt, wobei zwischen der Außenschicht 17, die eine elektrisch leitende Schicht 16 bildet, und der Innenschicht 18, die eine mechanisch tragende Schicht 14 bildet, eine Isolierschicht 15 als Zwischenschicht 19 angeordnet ist. Die Isolierschicht 15 trennt die elektrisch leitende Schicht 16 galvanisch von der mechanisch tragenden Schicht 14.

In diesem Zusammenhang wird darauf hingewiesen, dass die Begriffe "elektrisch leitende Schicht" und "mechanisch tragende Schicht" die bevorzugte bzw. hauptsächliche Funktion der jeweiligen Schichten wiedergeben. Dabei ist nicht ausgeschlossen, dass beispielsweise die mechanisch tragende Schicht auch elektrisch leitende Eigenschaften umfasst. Ebenso kann die elektrisch leitende Schicht mechanisch tragende Funktionen aufweisen. Wesentlich ist im Rahmen der Anmeldung, dass die entsprechenden Schichten jeweils eine Hauptfunktion aufweisen bzw. für eine Hauptfunktion angepasst sind, wobei die Hauptfunktion der elektrisch leitenden Schicht durch eine erhöhte Leitfähigkeit und die Hauptfunktion der mechanisch tragenden Schicht durch eine erhöhte Stabilität gekennzeichnet sind.

Bei dem Ausführungsbeispiel gemäß Fig. 4 ist die Zwischenschicht 19 als Isolierschicht 15 ausgebildet. Dadurch wird erreicht, dass sich die Stromführung in der Elektrode auf die Außenschicht 17, nämlich die elektrisch leitende Schicht 16 beschränkt. Ein direkter Ladungsaustausch zwischen der Außenschicht 17 und der Innenschicht 18 findet also nicht statt. Zwar können in der Innenschicht 18 bzw. der mechanisch tragenden Schicht 14 Ströme durch Induktion entstehen. Dieser Effekt ist jedoch vernachlässigbar, da die Ströme in der Elektrode entsprechend klein gewählt sind. Dies gilt insbesondere bei Verwendung der Elektrode zur Signalerfassung, wobei die elektrischen Potentiale innerhalb des menschlichen Körpers vergleichsweise klein sind. Überdies können induktive Effekte durch Wahl der entsprechenden Materialien, insbesondere für die mechanisch tragende Schicht 14 und die Isolierschicht 15, reduziert bzw. verhindert werden.

Es ist auch möglich, die Außenschicht 17 und die Innenschicht 18 als Pole der Elektrode auszubilden, die mit derselben oder mit unterschiedlichen Spannungen beaufschlagbar sind. Dazu sind die beiden Schichten 17, 18 jeweils mit einer gesonderten Stromversorgung verbunden.

Wie bei dem Ausführungsbeispiel gemäß Fig. 1 dargestellt, kann vorteilhaft vorgesehen sein, dass die Gitterstruktur 10 insgesamt elektrisch leitende Eigenschaften aufweist. Das Material der Gitterstruktur 10 kann also eine Doppelfunktion umfassen, nämlich einerseits ausreichend mechanisch tragende Funktionen bereitstellen, um die Elektrode bzw. die Gitterstruktur 10 effizient in einem Hohlorgan zu fixieren, und andererseits elektrisch leitende Eigenschaften bereitstellen, die eine elektrische Stimulierung von Körpergewebe bzw. eine elektrische Signalerfassung von elektrischen Zellpotentialen ermöglichen. Dabei ist nicht ausgeschlossen, dass ein Kontakt der elektrisch leitenden Schicht 16 bzw. der insgesamt elektrisch leitenden Gitterstruktur 10 mit Körperflüssigkeiten, insbesondere Blut zur Erhöhung der Biokompatibilität der Elektrode führt. Dabei kann die Thrombogenität gesenkt und die Neigung zur Endothelialisierung verbessert werden. Andererseits kann in bestimmten Anwendungen ein elektrischer Kontakt zwischen der Elektrode und Körperflüssigkeiten, insbesondere Blut, als nachteilig angesehen werden, so dass in diesen Fällen die Verwendung einer mehrschichtigen Gitterstruktur 10, vorteilhaft ist, wobei die elektrische Kontaktfläche zur Körperflüssigkeit bzw. zum Blut auf ein Minimum begrenzt ist. Die entsprechende Variante der Gitterstruktur 10 bzw. allgemeinen Elektrode wählt der Fachmann anhand der jeweiligen medizinischen Vorgaben.

Es ist ferner möglich, dass die Gitterstruktur 10 einen mehrschichtigen Aufbau aufweist, wobei eine Zwischenschicht 19 zwei elektrisch leitende Schichten 16 voneinander trennt. Den elektrisch leitenden Schichten 16 können unterschiedliche, insbesondere therapeutische, Funktionen zugeordnet sein. Beispielsweise kann die Außenschicht 17 als elektrisch leitende Schicht 16 ausgebildet sein, wobei die Außenschicht 17 mit einem elektrischen Signal beaufschlagt wird, mit dem eine elektrische Stimulation von Körperzellen, insbesondere Nervenzellen, erreicht wird. Die Innenschicht 18 kann hingegen eine elektrisch leitende Schicht 16 bilden, die derart mit elektrischen Signalen angesteuert wird, dass die Biokompatibilität der Elektrode erhöht ist. Konkret können die unterschiedlichen elektrischen Schichten 16, also die Außenschicht 17 und die Innenschicht 18, mit unterschiedlichen elektrischen Spannungen beaufschlagt werden. Weitere Parameter, die in einzelnen elektrischen Schichten 16 variierbar sind, sind die Stromstärke, die Pulsfrequenz bzw. allgemein der Stromverlauf.

Die Gitterstruktur 10 kann ferner mit wenigstens einem Medikament beladen sein. Mit anderen Worten kann die Gitterstruktur 10 eine medikamententragende Schicht umfassen. Vorzugsweise ist die medikamententragende Schicht direkt mit einer elektrisch leitenden Schicht 16 verbunden, so dass durch Beaufschlagung der elektrisch leitenden Schicht 16 mit einer Spannung bzw. allgemein einem elektrischen Signal zumindest Teile des Medikaments bzw. der therapeutisch wirksamen Substanz freisetzbar sind. Dabei kann das Medikament an der Oberfläche oder im Material vorgesehen sein.

Die mechanisch tragende Schicht 14 kann ein biodegradierbares Material, beispielsweise Magnesium oder eine Eisenlegierung umfassen. Die biodegradierbare mechanisch tragende Schicht 14 verleiht der Gitterstruktur 10 zunächst eine ausreichende mechanische Stabilität, so dass die Gitterstruktur 10 zur Stützung eines Körperhohlorgans A einsetzbar ist. Im zeitlichen Verlauf baut sich die biodegradierbare Schicht ab, wobei die Gitterstruktur 10 gleichzeitig von Endothelzellen eingefasst wird. Die vergleichsweise dünne elektrisch leitende Schicht 16 bleibt bestehen bzw. wird nicht abgebaut. Durch den Abbau der mechanisch tragenden Schicht 14 wird das Hohlorgan A mechanisch entlastet, so dass sich die ursprüngliche Compliance des Hohlorgans A wieder einstellt. Aufgrund der vergleichsweise dünnen Schichtdicke der elektrisch leitenden Schicht 16 wirkt sich die untergeordnete mechanische tragende Funktion der elektrisch leitenden Schicht 16 nur vernachlässigbar auf die Biomechanik des Hohlorgans A aus. Es ist auch möglich, dass die gesamte Elektrode biologisch abbaubar ist.

Das erfindungsgemäße Herstellungsverfahren, nämlich die Verwendung der physikalischen Gasphasenabscheidung, insbesondere eines PVD-Verfahrens bzw. Sputterverfahrens, ermöglicht besonders einfach die Herstellung eines mehrschichtigen Aufbaus der Gitterstruktur 10. Insbesondere können die unterschiedlichen Schichten der Gitterstruktur 10 in einem einzigen Verfahrensschritt hergestellt werden. Dabei erfolgt eine stoffschlüssige Verbindung zwischen den einzelnen Schichten. Mit anderen Worten wird die Gitterstruktur 10 mit dem erfindungsgemäßen Verfahren schichtweise aufgebaut. Im Unterschied zu herkömmlichen Verfahren zur Herstellung endovaskulärer Elektroden, wobei mehrere Bauteile bzw. Komponenten separat hergestellt und anschließend miteinander verbunden werden, ermöglicht das Verfahren der physikalischen Gasphasenabscheidung den Aufbau mehrerer Schichten in einem Verfahrensschritt, wobei die Gitterstruktur einstückig und schichtweise aufgebaut wird. Dabei versteht sich, dass die einzelnen funktional getrennten Schichten 14, 15, 16 selbst wiederum schichtweise aufgebaut sind und Laminate bilden. Dies ergibt sich aus der Herstellung der Schichten durch Abscheideverfahren, insbesondere durch Sputtern.

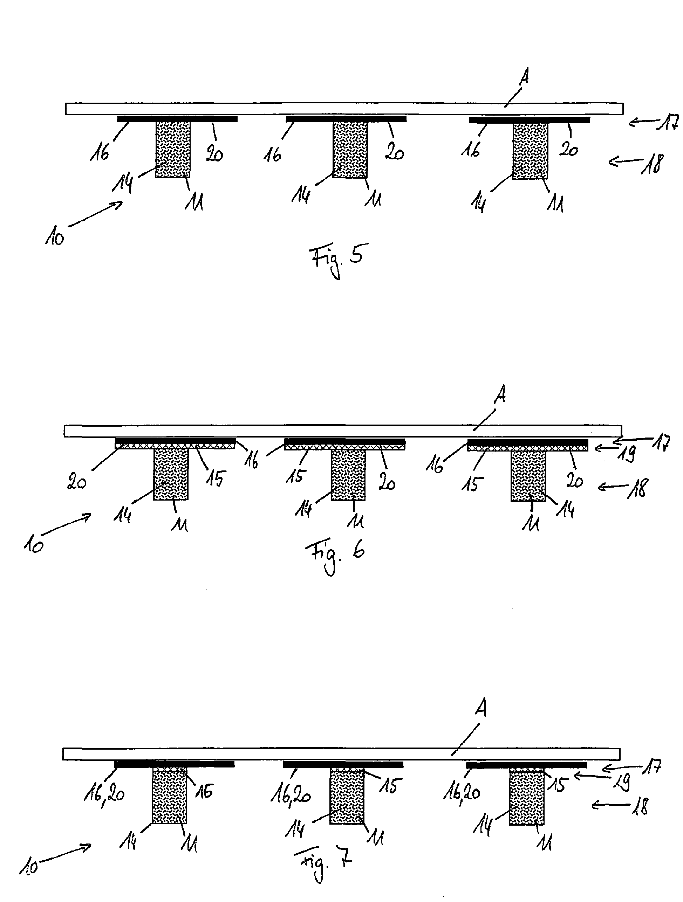

Um eine schonende Behandlung mit relativ niedrigen Stromdichten zu erreichen, ist die Kontaktfläche zwischen der Gitterstruktur 10 und dem Hohlorgan A vorzugsweise vergrößert. In den Fig. 5 bis 7 sind entsprechende Ausführungsbeispiele der Erfindung dargestellt, wobei die Gitterstege 11 eine Verbreiterung 20 umfassen. Insbesondere weist die elektrisch leitende Schicht 16 der Gitterstege 11 jeweils die Verbreiterung 20 auf. Die Verbreiterung 20 folgt dem Verlauf der Gitterstege und erstreckt sich in Längsrichtung der Gitterstege 11 derart, dass der mit dem Hohlorgan A in Berührung kommende Bereich der Gitterstege 11 breiter als der vom Hohlorgan A entfernte Bereich der Gitterstege 11, insbesondere die mechanisch tragende Schicht 14, ist. Im Querschnittsprofil weist der Gittersteg 11 mit der Verbreiterung 20 im Wesentlichen eine T-Form auf. Ein mögliches Verfahren zur Herstellung der Verbreiterung 20 ist in DE 10 2008 010 507 offenbart, die auf die Anmelderin zurückgeht und die Verbindung der Gitterstege 11 mit flexiblen Kontaktelementen durch Sputtern offenbart. Der Inhalt der DE 10 2008 010 507 wird durch Verweis vollumfänglich in die vorliegende Anmeldung aufgenommen.

Fig. 5 zeigt einen Längsschnitt durch die Gitterstruktur 10 einer Elektrode, wobei die Gitterstege 11 einen zweischichtigen Aufbau gemäß Fig. 3 aufweisen mit einer als mechanisch tragende Schicht 14 ausgebildeten Innenschicht 18 und einer als elektrisch leitende Schicht 16 ausgebildeten Außenschicht 17, die eine Verbreiterung 20 umfasst. Das Ausführungsbeispiel gemäß Fig. 5 unterscheidet sich also von dem Ausführungsbeispiel gemäß Fig. 3 dadurch, dass die elektrisch leitende Schicht 16 verbreitert ist. Dadurch wird die Kontaktfläche zum Hohlorgan A vergrößert. Die Verbreiterung 20 und die mechanisch tragende Schicht 14 können dasselbe Material umfassen. Dabei umfassen die Verbreiterung 20 und die mechanisch tragende Schicht 14 vorzugsweise eine Nickel-Titan-Legierung, insbesondere Nitinol bzw. allgemein ein pseudoelastisches Material. Die Verwendung von pseudoelastischen Materialien für die Verbreiterung 20 ermöglicht ein sehr flexibles Verhalten der Verbreiterung 20, so dass mehrere auf dem Umfang der Gitterstruktur 10 angeordnete Verbreiterungen 20 beim Komprimieren der Gitterstruktur 10 einander ausweichen oder überlappen oder aufeinander gleiten oder sich elastisch verformen können. Die Komprimierbarkeit bzw. Crimpbarkeit oder der Grad der maximal möglichen Komprimierung wird somit erhöht. Ferner ermöglicht eine erhöhte Flexibilität der Verbreiterung 20 eine verbesserte Anlage der Gitterstruktur 10 an das Hohlorgan A.

Gitterstrukturen mit Verbreiterungen bzw. Abdeckungen und Verfahren zu deren Herstellung sind in DE 10 2008 010 507, DE 10 2009 060 228, DE 10 2009 060 280 beschrieben, die durch Verweis vollumfänglich in die vorliegende Anmeldung aufgenommen werden.

Die Verbreiterung 20 und die mechanisch tragende Schicht 14 können unterschiedliche Materialien umfassen. Beispielsweise kann die mechanisch tragende Schicht 14 ein Formgedächtnismaterial und die Verbreiterung 20 ein Material mit erhöhter Leitfähigkeit, beispielsweise Gold, aufweisen. Die elektrischen Eigenschaften der Gitterstruktur 10 bzw. allgemein der Elektrode können auf diese Weise verbessert werden.

Die Verbreiterung 20 kann überdies mehrere Schichten aufweisen. Beispielsweise kann die Verbreiterung 20, wie in Fig. 6 dargestellt, eine elektrisch leitende Schicht 16 als Außenschicht 17 und eine Isolierschicht 15 aufweisen, die radial weiter innen angeordnet ist. Die Isolierschicht 15 kann insbesondere analog zu dem Ausführungsbeispiel gemäß Fig. 4 als Zwischenschicht 19 ausgebildet sein, die die elektrisch leitende Schicht 16 (Außenschicht 17) von einer mechanisch tragenden Schicht 14 (Innenschicht 18) trennt. Die Isolierschicht 15 erstreckt sich bei dem Ausführungsbeispiel gemäß Fig. 6 über die gesamte Breite der Verbreiterung 20 bzw. bildet einen Teil der Verbreiterung 20. Die mechanisch tragende Schicht 14 ist hingegen schmaler ausgebildet, so dass die Gitterstruktur 10 insgesamt eine gute Crimpbarkeit aufweist. Vorzugsweise ist die Verbreiterung 20, insbesondere die elektrisch leitende Schicht 16 und die Isolierschicht 15 derart dünn und flexibel ausgebildet, dass die Crimpbarkeit der Gitterstruktur 10 nicht oder zumindest nur unwesentlich beeinträchtigt wird.

Die Zwischenschicht 19 gemäß Fig. 6 kann ein Formgedächtnismaterial, beispielsweise eine Nickel-Titan-Legierung umfassen. Die Zwischenschicht 19 bestimmt dabei die Flexibilität der Verbreiterung 20. Die elektrisch leitende Schicht 16 der Verbreiterung 20 kann hingegen ein Material mit erhöhten elektrischen Leitfähigkeitseigenschaften, beispielsweise Gold, aufweisen. Vorzugsweise umfasst die elektrisch leitende Schicht 16 eine Schichtdicke, die höchstens der Schichtdicke der Zwischenschicht 19 entspricht. Insbesondere beträgt die Schichtdicke der elektrisch leitenden Schicht 16 bzw. Außenschicht 17 höchstens 100%, insbesondere höchstens 80%, insbesondere höchstens 40%, insbesondere höchstens 20%, insbesondere höchstens 10%, insbesondere höchstens 5%, insbesondere höchstens 2%, bezogen auf die Schichtdicke der Zwischenschicht 19 oder der Innenschicht 18. Die Zwischenschicht 19 kann auch eine Isolierschicht 15 bilden bzw. ein isolierendes Material umfassen. Die Außenschicht 17 kann eine Formgedächtnislegierung wie beispielsweise Nitinol aufweisen. Die Verbreiterung 20 ist nicht auf einen ein- oder zweischichtigen Aufbau beschränkt. Vielmehr kann die Verbreiterung 20 auch drei oder mehr Schichten umfassen.

Bei dem Ausführungsbeispiel gemäß Fig. 6 erstreckt sich die Zwischenschicht 19 über die gesamte Breite der Verbreiterung 20. Die Zwischenschicht 19 ist also der Verbreiterung 20 zugeordnet. Alternativ kann vorgesehen sein, dass die Zwischenschicht 19 der Innenschicht 18 bzw. mechanisch tragenden Schicht 14 zugeordnet ist, wie in Fig. 7 dargestellt. Mit anderen Worten entspricht die Breite der Zwischenschicht 19 bei dem Ausführungsbeispiel gemäß Fig. 7 der Breite der mechanisch tragenden Schicht 14. Eine weitere Betrachtungsweise besteht darin, die Zwischenschicht 19 gemäß Fig. 7 dem Gittersteg 11 zuzuordnen, der zusätzlich eine Verbreiterung 20 umfasst, die mit der Zwischenschicht 19 verbunden ist. Im Rahmen dieser Betrachtungsweise ist auszuführen, dass sowohl der Gittersteg 11, als auch die Verbreiterung 20 jeweils einen mehrschichtigen Aufbau aufweisen kann. Dabei wird darauf hingewiesen, dass die Verbreiterung 20 und der Gittersteg 11 integral miteinander verbunden sind. Insbesondere wird die Verbreiterung 20 durch den schichtweisen Aufbau der Gitterstruktur 10 mittels des Gasphasenabscheideverfahrens einteilig mit dem Gittersteg 11 ausgebildet.

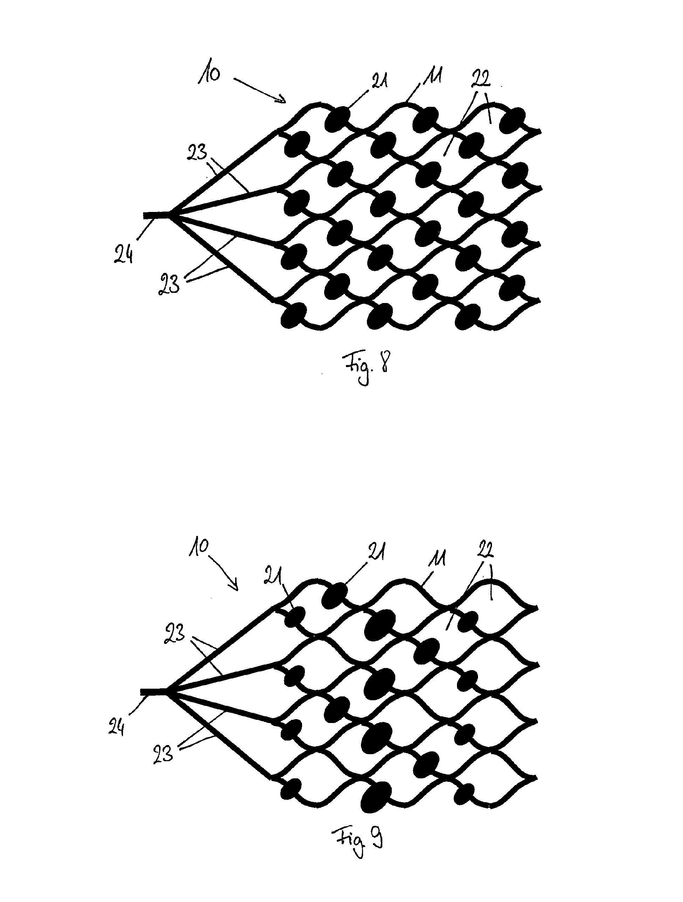

In Fig. 8 ist ein Ausführungsbeispiel der Elektrode dargestellt, wobei die Gitterstruktur 10 Gitterstege 11 umfasst, die eine Abdeckung 21 aufweisen. Die Abdeckung 21 kann alternativ oder zusätzlich zu einer Verbreiterung 20 vorgesehen sein. Die Abdeckung 21 ist mit einem Gittersteg 11 verbunden und bildet zumindest einen Teil der Außenfläche des jeweiligen Gitterstegs 11. Dabei weicht die Längsrichtung der Abdeckung 21 von der Längsrichtung des jeweiligen Gitterstegs 11 ab. Konkret erstreckt sich die Abdeckung 21 jeweils in die abgrenzende Zelle 22 und deckt diese zumindest teilweise ab. Bei dem Ausführungsbeispiel gemäß Fig. 8 verläuft die Längsrichtung der Abdeckung 21 quer zur Längsrichtung des jeweiligen Gitterstegs 11. Dabei können jeder Zelle 22 ein oder mehrere Abdeckungen 21 zugeordnet sein. Gemäß Fig. 8 sind jeder Zelle 22 jeweils zwei Abdeckungen 22 zugeordnet. In Fig. 8 ist ferner erkennbar, dass nicht alle Gitterstege 11 eine Abdeckung 21 aufweisen. Vielmehr umfasst die Gitterstruktur 10 Gitterstege 11, die mit einer Abdeckung 21 versehen sind und weitere Gitterstege 11, die abdeckungsfrei ausgebildet sind. Konkret ist bei dem Ausführungsbeispiel gemäß Fig. 8 vorgesehen, jeden zweiten Gittersteg 11 mit der Abdeckung 21 auszustatten.

Die Abdeckungen 21 gemäß Fig. 8 sind jeweils unter einem Winkel von 90 Grad zum jeweiligen Gittersteg 11, jeweils bezogen auf die Längsrichtung der Abdeckung 21 bzw. des Gitterstegs 11, angeordnet. Eine andere Ausrichtung der Abdeckung 21 ist möglich. Die Abdeckung 21 ist ferner oval ausgebildet, wobei weitere bzw. andere geometrische Formen, beispielsweise kreisförmige Abdeckungen 21, möglich sind. Dasselbe gilt für die Größe der Abdeckung 21, die derart angepasst sein kann, dass die Abdeckung 21 die jeweilige Zelle mit verschiedenen Abdeckungsgraden entdeckt. Beispielsweise kann die Abdeckung 21 die jeweilige Zelle 22 derart überdecken, dass sich benachbarte Abdeckungen 21 im expandierten Zustand der Gitterstruktur 10 überlappen. Die Abdeckung 21 und der zugehörige Gittersteg 11 können aus dem selben oder aus unterschiedlichen Materialien bestehen. Die Abdeckung 21 und der zugehörige Gittersteg 11 können einteilig ausgebildet sein. In diesem Zusammenhang wird auf die Erläuterungen zu den Verbreiterungen 20 verwiesen.

Der Unterschied zwischen einer Abdeckung 21 und einer Verbreiterung 20 besteht darin, dass die Verbreiterung 20 dem Verlauf des Gittersteges 11 folgt, also im wesentlichen parallel zum Gittersteg 11 angeordnet ist. Die Abdeckung weist eine Längserstreckung auf, die vom Verlauf des Gittersteges 11 abweicht. Zwischenformen sind möglich, die Abdeckungen 21 und Verbreiterungen 20 kombinieren, bspw. Verbreiterungen 20, die sich seitlich bezogen auf den zugehörigen Gittersteg 11 auswölben, wobei sich die Auswölbung in eine Zelle erstreckt. Die Abdeckungen 21 und Verbreiterungen 20 bilden eine Außenschicht 17 auf der tragenden Schicht 14. Sie können aus demselben Material wie die tragende Schicht 14 gebildet sein, wobei im Fall eines leitfähigen Materials eine Isolierschicht 15 zwischen den Abdeckungen 21 und Verbreiterungen 20 einerseits und der tragenden Schicht 14 andererseits angeordnet ist.

In Fig. 8 ist zur Verbindung der Elektrode mit der Stromversorgung bzw. der Stromzufuhr eine zentrale Versorgungsleitung 24 dargestellt, die mehrere einzelne Versorgungsleitungen 23 bündelt. Die zentrale Versorgungsleitung 24 kann zentrale Funktionen übernehmen und beispielsweise als Betätigungsmittel für die Zufuhr oder das Wiedereinziehen der Elektrode ausgebildet sein. Konkret kann die zentrale Versorgungsleitung 24 einen Führungsdraht bilden. Die einzelnen Versorgungsleitungen 23 sind mit den Gitterstegen der Gitterstruktur 10 verbunden. Die Versorgungsleitungen 23 können mit einzelnen Schichten, insbesondere mit den bzw. der leitenden Schicht 16 verbunden sein. Bei dem Ausführungsbeispiel gemäß Fig. 8 sind vier Versorgungsleitungen 23 vorgesehen. Eine andere Art der Versorgungsleitungen 23 ist möglich. Insbesondere kann auch vorgesehen sein, dass die zentrale Versorgungseinleitung 24 direkt mit der Gitterstruktur 10 verbunden ist.

Im Allgemeinen ist die Elektrode mit einer Stromversorgung bzw. Stromzufuhr gekoppelt oder mit einer Stromzufuhr koppelbar. Dazu kann die Elektrode bzw. die Gitterstruktur 10 durch eine Leitung mit einer (nicht dargestellten) Stromzufuhr verbunden sein. Die Stromzufuhr der Elektrode kann als Modul zusammen mit einem Impulsgeber und einer Steuerelektronik ausgebildet sein. Die Verbindung zwischen der Stromzufuhr und der Elektrode kann einerseits durch eine elektrische Leitung, insbesondere einen Draht oder die zentrale Versorgungsleitung 24, erfolgen. Es ist möglich, lebenslang oder zumindest während der Dauer der Behandlung funktionsfähige Batterien zu verwenden.

Andererseits ist es möglich, dass die Verbindung zwischen der Stromzufuhr und der Elektrode drahtlos erfolgt, beispielsweise durch Induktion eines elektrischen Stroms in der Gitterstruktur 10 über ein transkutanes Energieübertragungssystem (TET-System). Dazu weist die Elektrode ein Mittel zur Kopplung mit einer Stromzufuhr bzw. ein Mittel zur Stromübertragung, insbesondere zur induktiven Stromübertragung auf, das mit der Gitterstruktur, insbesondere mit der elektrisch leitenden Schicht 16 verbunden ist. Dabei umfasst die Elektrode einen Empfänger, insbesondere eine Empfangsspule, die zumindest mit der elektrisch leitenden Schicht 16 der Gitterstruktur 10 elektrisch gekoppelt ist. Der Empfänger bzw. die Empfangsspule kann durch einen extrakorporalen Sender bzw. eine extrakorporale Sendespule angeregt werden. Auf diese Weise kann in der Empfangsspule bzw. im Empfänger eine elektrische Spannung induziert werden. Zusätzlich zu der Empfangsspule können ein Impulsgeber und eine Steuerelektronik vorgesehen sein, die mit der Gitterstruktur 10 verbunden ist. Der Impulsgeber und die Steuerelektronik können auch extrakorporal angeordnet sein und drahtlos, insbesondere per Funk, mit der Empfangsspule bzw. einem geeigneten Empfänger kommunizieren. Außerdem ist es möglich, eine nachladbare Batterie bzw. einen Kondensator, in die Elektrode zu integrieren, die über eine Ladevorrichtung durch die Empfangsspule wiederaufladbar ist. Derartige Systeme zur transkutanen Energieübertragung sind an sich bekannt und können zusammen mit der Erfindung eingesetzt werden. Es wird sowohl die Elektrode an sich mit dem Mittel zur Kopplung mit einer Stromzufuhr offenbart als auch das vorbeschriebene System, mit einer derartigen Elektrode.

Fig. 9 zeigt ein weiteres Ausführungsbeispiel der Elektrode, wobei sich das Ausführungsbeispiel gemäß Fig. 9 von dem Ausführungsbeispiel gemäß Fig. 8 dadurch unterscheidet, dass die Abdeckungen 21 inhomogen über die Gitterstruktur 10 verteilt sind. Die Abdeckungen 21 weisen überdies unterschiedliche Größen auf. Insgesamt kann vorgesehen sein, dass die Dichte der Abdeckungen 21 bzw. die Kontaktfläche der Abdeckungen 21 mit dem Hohlorgan A über die Gesamtfläche der Gitterstruktur 10 variiert. Durch die unterschiedlichen geometrischen Abmessungen der einzelnen Abdeckungen 21 und die variable Verteilung der Abdeckungen 21 über die Gitterstruktur 10 kann das therapeutisch wirksame bzw. erzeugte elektrische Feld im Wesentlichen beliebig eingestellt werden. Die Einstellung des elektrischen Feldes, also die geometrische Anordnung und Verteilung der Abdeckungen 21 wählt der Fachmann anhand medizinischer Vorgaben, insbesondere unter Berücksichtigung der zu behandelnden Erkrankung.

Für beide Ausführungsbeispiele gemäß Fig. 8 und 9 gilt, dass die Außenschicht 17 der Gitterstege 11 sowohl elektrisch leitend als auch isolierend ausgebildet sein kann. Insbesondere kann vorgesehen sein, dass die Außenschicht 17 eine elektrisch leitende Schicht 16 bildet, so dass die Gesamtfläche der Gitterstruktur 10 einschließlich der Abdeckungen 21 zur elektrischen Kopplung der Elektrode mit dem Hohlorgan A dient. Alternativ können die Gitterstege 11 als Außenschicht 17 eine Isolierschicht 15 umfassen, so dass der elektrische Kontakt zwischen der Elektrode und dem Hohlorgan A ausschließlich durch die Abdeckungen 21 erfolgt. Die Abdeckungen 21 sind dazu mit einer unterhalb der Isolierschicht 15 angeordneten elektrisch leitenden Schicht 16 verbunden. Auf diese Weise sind die Abdeckungen 21 untereinander elektrisch gekoppelt, wobei die Gitterstege 11 keine direkte elektrische Verbindung mit dem Hohlorgan A eingehen. Es ist auch möglich, die Abdeckung 21 und die Außenschicht 17 in einem Bereich der Gitterstege 11 um die Abdeckung 21 herum elektrisch leitend auszubilden. Insgesamt besteht eine Vielzahl von Designmöglichkeiten, die eine Modulation des mit der Elektrode erzeugbaren elektrischen Feldes erlauben.

Die Herstellung der Abdeckung 21 oder Verbreiterung 20 erfolgt vorzugsweise durch ein strukturierendes Verfahren, beispielsweise lithographisches Ätzen. Dabei wird die Gitterstruktur 10 oder zumindest einzelne Schichten der Gitterstruktur, wie bspw. die Isolierschicht oder die leitende Schicht 16, zunächst durch den physikalischen Gasphasenabscheideprozess schichtweise aufgebaut und anschließend die Zellen 22 durch das Ätzverfahren freigelegt, wobei durch eine entsprechende lithographische Maske zuvor die Form und Abmessung der Abdeckungen 21 bzw. Verbreiterungen 20 und Gitterstege 11 festgelegt wurde. Dabei kann auch vorgesehen sein, dass unterhalb der Abdeckungen 21 abschnittsweise eine Isolierschicht 15 vorgesehen ist. Zumindest in einem Teilbereich des Gitterstegs 11 und der Abdeckung 21 ist eine elektrische Verbindung zwischen einer elektrisch leitenden Schicht 16 und der Abdeckung 21 vorgesehen. Es ist auch möglich, dass die leitende Schicht 16 und die Abdeckung 21 in derselben Ebene angeordnet sind und die elektrische Verbindung an der Stößstelle zwischen der leitenden Schicht 16 und der Abdeckung 21 ausgebildet ist. Die Abdeckung 21 kann analog zur Verbreiterung 20 mehrschichtig aufgebaut sein. Vorzugsweise umfasst die Abdeckung 21 wenigstens eine elektrisch leitende Schicht 16, die eine Außenschicht 17 der Gitterstruktur 10 bildet, also einen elektrischen Kontakt mit dem Hohlorgan A herstellt. Die Abdeckung 21 ist elektrisch mit weiteren Abdeckungen 21 gekoppelt.

Die Abdeckung 21 kann ein elastisches Material umfassen. Beim Komprimieren der Gitterstruktur 10 gleiten benachbarte Abdeckungen 21 übereinander und überlappen, um die Komprimierung der Gitterstruktur 10 nicht zu behindern.

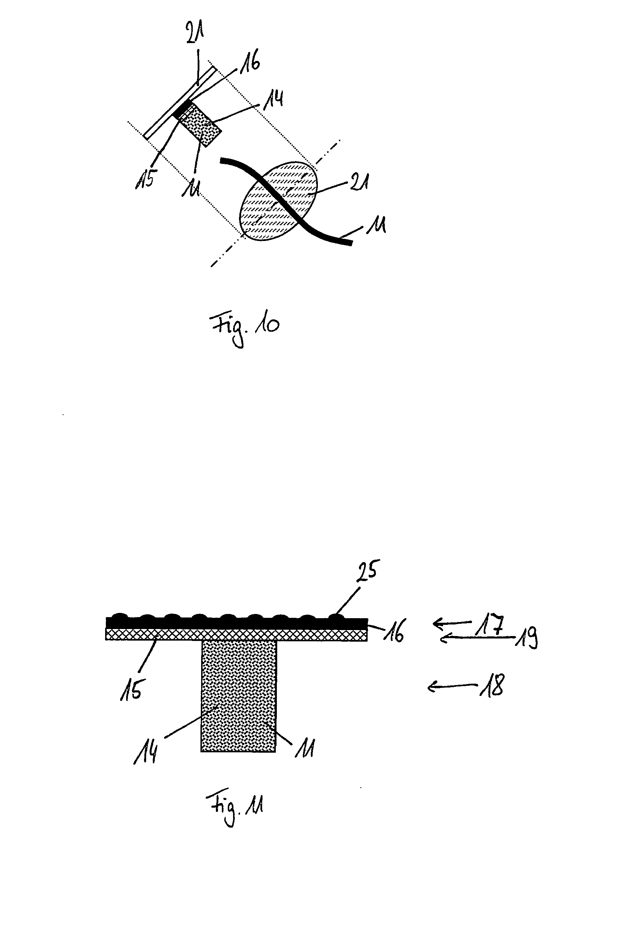

In Fig. 10 ist ein möglicher Schichtaufbau eines Gitterstegs 11 mit einer Abdeckung 21 dargestellt. Dabei umfasst der Gittersteg 11 eine mechanisch tragende Schicht 14, die die Innenschicht 18 der Gitterstruktur 10 bildet. Auf der mechanisch tragenden Schicht 14 ist eine Isolierschicht 15 angeordnet, die eine Zwischenschicht 19 bildet. Auf der Zwischenschicht 19 bzw. Isolierschicht 15 ist ferner eine elektrisch leitende Schicht 16 angeordnet, die gleichzeitig elastische Eigenschaften aufweist. Die elektrisch leitende Schicht 16 bildet eine zweite Zwischenschicht 19. Die Außenschicht 17 des Gitterstegs 11 wird durch die Abdeckung 21 gebildet, die ebenfalls ein elektrisch leitendes Material umfasst. Die elektrische Kontaktierung zwischen mehreren Abdeckungen 21 erfolgt durch die elektrisch leitende Schicht 16.

Die Außenschicht 17, insbesondere die Abdeckung 21 oder die Verbreiterung 20, kann zur Erhöhung der elektrischen Kontaktfläche strukturiert sein. Beispielsweise kann die Außenschicht 17 Rillen, Aussparungen, Vorsprünge oder weitere Strukturen aufweisen, wodurch die elektrische Gesamtkontaktfläche vergrößert wird. Eine derartige Strukturierung 25 ist beispielhaft in Fig. 11 dargestellt. Dabei weist die Gitterstruktur 10 gemäß Fig. 11 einen Gittersteg 11 auf, der eine mechanisch tragende Schicht 14 als Innenschicht 18 umfasst. Auf die mechanisch tragende Schicht 14 folgt eine Abdeckung 21 bzw. Verbreiterung 20, die eine Isolierschicht 15 als Zwischenschicht 19 aufweist. Die Außenschicht 17 der Gitterstruktur 10 ist Teil der Abdeckung 21 bzw. Verbreiterung 20 und umfasst eine Strukturierung 25. Die Strukturierung 25 kann einfach durch das physikalische Gasphasenabscheideverfahren hergestellt werden. Dabei wird das Substrat mit einer entsprechenden Negativform strukturiert, so dass sich bei der Gasphasenabscheidung, insbesondere beim Sputtern, die strukturierte Oberfläche einstellt. Ferner kann die Strukturierung 25 durch ein Ätzverfahren im Anschluss an die physikalische Gasphasenabscheidung hergestellt werden. Mit beiden Herstellungsverfahren sind sowohl runde Formen als auch scharfe Kanten der Strukturierung 25 einstellbar.

Im Hinblick auf die Verbindung der Elektrode mit einer Stromzufuhr ist vorgesehen, dass die zentrale Versorgungsleitung 24 eine Nickel-Titan-Legierung umfasst, insbesondere Nitinol. Vorzugsweise ist die zentrale Versorgungsleitung 24 als Führungsdraht gestaltet. Konkret kann die zentrale Versorgungsleitung 24 eine Seele, die vorzugsweise Nitinol aufweist, und einen Kunststoffschlauch umfassen, der die Seele umgibt. Der Kunststoffschlauch, dessen Material beispielsweise PTFE oder PVC aufweisen kann, nimmt eine Doppelfunktion wahr. Einerseits dient der Kunststoffschlauch zur Isolierung der stromführenden Seele der zentralen Versorgungsleitung 24. Andererseits bietet der Kunststoffschlauch eine flexible Stabilisierung der Seele.

Bezüglich der Gitterstruktur 10 können unterschiedliche Anordnungen der zentralen Versorgungsleitung 24 vorgesehen sein. Beispielsweise kann die zentrale Versorgungsleitung 24 bezogen auf die expandierte Gitterstruktur 10 fluchtend mit der Längsachse der Gitterstruktur 10 angeordnet sein. Alternativ kann die zentrale Versorgungsleitung 24 fluchtend mit dem Außenumfang der Gitterstruktur 10 angeordnet sein. Durch die seitliche bzw. in fluchtender Anordnung zum Außenumfang der Gitterstruktur 10 angeordnete zentrale Versorgungsleitung wird erreicht, dass beim endovaskulären Einsatz in Blutgefäßen der Blutfluss durch die zentrale Versorgungsleitung 24 im Wesentlichen nicht behindert wird. Die zentrale Versorgungsleitung 24 kann mit der Gitterstruktur 10 fest verbunden sein. Alternativ kann die zentrale Versorgungsleitung 24 mit der Gitterstruktur 10 lösbar bzw. entkoppelbar verbunden sein. Die letztgenannte Variante eignet sich insbesondere bei Verwendung der Gitterstruktur 10 als Dauerimplantat. Es ist auch möglich, dass die Gitterstruktur 10 frei von einer zentralen Versorgungsleitung 24 ist. Eine Elektrode mit einer Gitterstruktur 10, die keine zentrale Versorgungsleitung 24 aufweist, wird im Rahmen der Anmeldung ebenfalls offenbart und beansprucht. Dabei handelt es sich um eine Elektrode mit einem Mittel zur induktiven Stromübertragung.

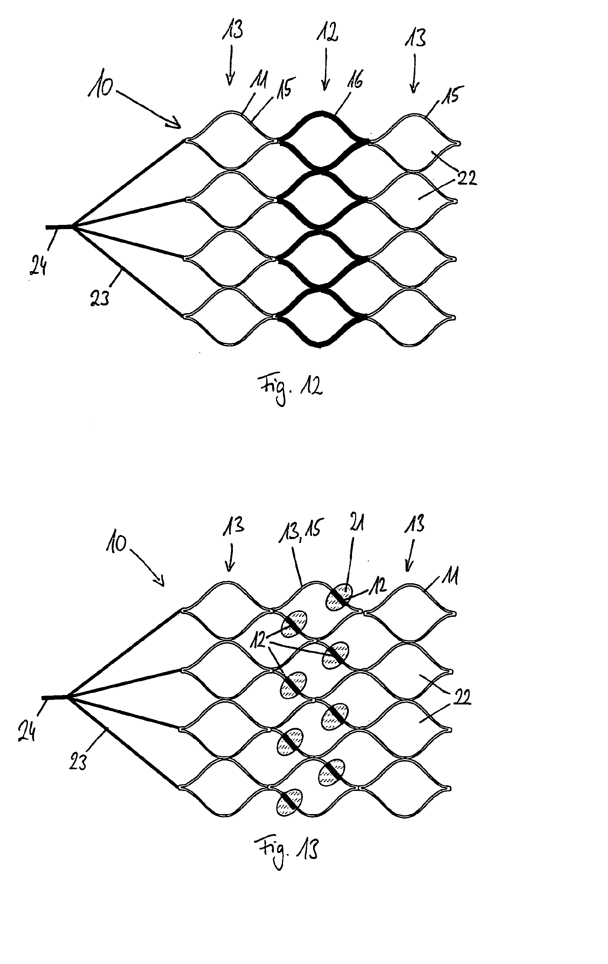

Es kann zweckmäßig sein, wenn Bereiche bzw. Abschnitte der Gitterstruktur 10 keine elektrisch leitende Außenschicht 17 umfassen. Auf diese Weise können gezielte Bereiche innerhalb eines Hohlorgans A mit elektrischen Signalen beaufschlagt werden bzw. in gezielten Bereichen des Hohlorgans A elektrische Signale erfasst werden. Ein derartiges Ausführungsbeispiel zeigt Fig. 12, wobei die Gitterstruktur 10 zwei elektrisch isolierte Bereiche 13 und einen dazwischen angeordneten elektrisch leitenden Bereich 12 umfasst. Die elektrisch isolierten Bereich 13 unterscheiden sich vom elektrisch leitenden Bereich 12 dadurch, dass in den elektrisch isolierten Bereichen 13 der Gitterstruktur 10 die Außenschicht 17 als Isolierschicht 15 ausgebildet ist. Das schließt nicht aus, dass unterhalb der Außenschicht 17 elektrisch leitende Schichten 16 angeordnet sind, die den elektrisch leitenden Bereich 12 mit der Stromzufuhr und/oder weiteren elektrisch leitenden Bereichen 12 verbinden. Die Herstellung des elektrisch leitenden Bereichs 12 und der elektrisch isolierten Bereiche 13 kann beispielsweise dadurch erfolgen, dass die Gitterstruktur 10 zunächst durch das physikalische Gasphasenabscheideverfahren schichtweise aufgebaut wird, wobei die oberste Schicht, insbesondere eine Außenschicht 17, als Isolierschicht 15 hergestellt wird. Nachfolgend wird durch ein lithographisches Ätzverfahren die Isolierschicht 15 bereichsweise abgetragen, so dass eine darunterliegende elektrisch leitende Schicht 16 freigelegt wird. Die freigelegte elektrisch leitende Schicht 16 bildet den elektrisch leitenden Bereich 12, wogegen der durch das Ätzverfahren nicht abgetragene Bereich der Isolierschicht 15 den elektrisch isolierten Bereich 13 bildet. Es ist möglich, die Isolierschicht 15 und die leitende Schicht auf gleicher Höhe, d.h. in derselben Ebene abzuscheiden. Es ist ferner möglich, die Isolierschicht 15 durch PVD-Verfahren aufzubringen und zur Bildung der leitenden Schicht 16 die Isolierschicht bereichsweise durch Ätzen zu entfernen.

Der elektrisch leitende Bereich 12 kann ganze Zellen 22 umfassen, wie in Fig. 12 dargestellt.

Es ist auch möglich, dass der elektrisch leitende Bereich 12 Stegabschnitte der Gitterstege 11 umfasst, wie in Fig. 13 dargestellt. Dabei sind die Gitterstege 11 im Wesentlichen mit einer als Isolierschicht 15 ausgebildeten Außenschicht 17 versehen. Ein Teil der Gitterstege 11 umfasst jeweils eine Abdeckung 21, wobei in dem Bereich des Gitterstegs 11 unterhalb der Abdeckung 21 jeweils ein elektrisch leitender Bereich 12 vorgesehen ist. Mit anderen Worten sind die Abdeckungen 21 in elektrisch leitenden Bereichen 12 der Gitterstege 11 angeordnet. Die Bereiche der Gitterstege 11, die außerhalb der Abdeckungen 21 angeordnet sind, bilden den elektrisch isolierten Bereich 13.

Die Elektrode für endovaskuläre medizinische Anwendungen eignet sich insbesondere zur Stimulation von Nerven, die sich in unmittelbarer Nähe von Hohlräumen bzw. Hohlorganen A und Gefäßen befinden. Die endovaskuläre Neuromodulation kann zur Behandlung von Gefäßkrankheiten wie hämorrhagischen und ischämischen Schlaganfall durch Einfluss auf Kreislaufparameter wie Blutdruck und Blutfluss, insbesondere durch Gefäßausweitung eingesetzt werden.

Dabei kann es sich um große oder kleine Gefäße handeln, z. B. Herzkranzgefäße oder um intrakranielle Gefäße und zwar sowohl um Arterien als auch um Venen, bspw. die Carotis Arterie oder die Jugularvene. Die Gitterstruktur 10 wird durch die als Führungsdraht ausgebildete zentrale Versorgungsleitung 24 über den Mikrokatheter 26 in das Blutgefäß geführt und dort aus dem Mikrokatheter 26 freigesetzt. Dadurch expandiert die Gitterstruktur 10 selbsttätig. Über die zentrale Versorgungsleitung 24 wird ferner ein gepulster Strom in die Gitterstruktur 10 geleitet, wodurch eine elektrische Stimulation der Nervenfasern bewirkt wird. Die elektrische Stimulation der Nervenfasern bewirkt eine Erweiterung der Blutgefäße, sodass Kreislaufstörungen, wie z. B. Durchflussstörungen oder Blutdruckstörungen behandelbar sind.

Die Elektrode bzw. Gitterstruktur 10 ist vorzugsweise wiedereinziehbar ausgebildet. Dazu kann ein axiales Ende der Gitterstruktur 10 eine schräge glatte Kante umfassen, so dass beim Vorschieben des Mikrokatheters 26 über die zentrale Versorgungsleitung 24 eine Komprimierung der Gitterstruktur 10 erfolgt, sobald die Spitze des Mikrokatheters 26 entlang der schrägen Abschlusskante der Gitterstruktur 10 gleitet.

Die Fig. 14 a und 14 b zeigen den Feldlinienverlauf des elektrischen Feldes, das von der Gitterstruktur 10 der Elektrode ausgeht, die mit einer nicht dargestellten, extrakorporal angeordneten zweiten Elektrode zusammenwirkt. Im Umfeld des Hohlorgans A verlaufen Nervenfasern einerseits parallel zum Hohlorgan A und andererseits orthogonal dazu. Die Elektrode, insbesondere Stentelektrode, stellt im Wesentlichen eine zylinderförmige Elektrode dar, wobei die Feldlinien B des elektrischen Feldes im Wesentlichen senkrecht zur Oberfläche bzw. zum Außenumfang der Gitterstruktur 10 verlaufen. Dadurch wird bewirkt, dass die orthogonal zur Achse der Gitterstruktur 10, also orthogonal zum Hohlorgan A verlaufende Nervenfasern C stimuliert werden. Die parallel zum Hohlorgan A verlaufenden Nervenfasern D erfahren im Wesentlichen keine Stimulation durch das elektrische Feld der Elektrode.

Vorzugsweise ist die Elektrode Teil eines Systems, das zusätzlich eine Stromversorgung, einen Impulsgeber, eine Steuerelektronik und gegebenenfalls eine Gegenelektrode E umfasst. Die Gegenelektrode E kann analog zur Elektrode ausgebildet sein. Alternativ kann die Gegenelektrode E eine extrakorporale Elektrode sein, die außerhalb des Körpers auf der Haut des Patienten angeordnet ist. Die weitere Elektrode bzw. Gegenelektrode kann auch durch die Spitze des Mikrokatheters 26 oder eines Führungsdrahtes gebildet sein. Die elektrische Kontaktierung zwischen der Gegenelektrode E und einer Stromzufuhr erfolgt über eine Leitung, die in der Wandung des Katheters zwischen zwei Kunststoffschichten angeordnet ist. Die Leitung kann ein Draht sein, der sich in axialer Richtung entlang des Mikrokatheters erstreckt. Alternativ kann ein vorhandener Coil oder ein Geflecht, das den Mikrokatheter stützt, als Leitung verwendet werden. Im Zusammenhang mit einer Gegenelektrode eignet sich das System besonders zur Signalerfassung elektrischer Zellaktivitäten. Durch Vergleichsmessung zwischen der Elektrode und der Gegenelektrode E können elektrische Potentiale innerhalb des Körpers gemessen und für weitere therapeutische Maßnahmen eingesetzt werden. Insofern ist das System bzw. die Elektrode als Sensor einsetzbar. Vorzugsweise kann das System bzw. die Elektrode zur Erfassung von Nervensignalen genutzt werden. Es ist möglich, dass das System einerseits zur Stimulation, also zur Abgabe elektrischer Signale in das Körpergewebe, und andererseits zur Signalerfassung, also zur Ermittlung elektrischer Signale aus dem Körpergewebe, verwendet wird. Zwischen den beiden Funktionen (Stimulation und Signalerfassung) kann manuell oder automatisch umgeschaltet werden.

Weitere Anwendungsgebiete der Elektrode für endovaskuläre medizinische Anwendungen betreffen die Behandlung von Parkinson, Epilepsie, Depression, Zwangsverhalten bzw. allgemein therapeutische Verfahren im Hinblick auf die tiefe Hirnstimulation. Ferner ist eine Anwendung der Elektrode im Zusammenhang mit Bluthochdruck (Hypertonie) beispielsweise in Nierengefäßen oder Halsschlagadern, denkbar. Die Elektrode ist nicht nur in Blutgefäßen, sondern auch in weiteren Körperhohlräumen, beispielsweise den Harnleitern, möglich.

Die Gitterstruktur 10 umfasst vorzugsweise einen Querschnittsdurchmesser im Herstellzustand zwischen 1 mm und 15 mm, insbesondere zwischen 2 mm und 10 mm, insbesondere zwischen 3 mm und 8 mm.

Das Verhältnis zwischen einem Bereich der Zelle 22, der durch die Abdeckung 21 abgedeckt ist und einen Bereich der Zelle 22, der frei durchgängig ist, beträgt vorzugsweise wenigstens 10%, insbesondere wenigstens 20%, insbesondere wenigstens 50%, insbesondere wenigstens 70%, insbesondere wenigstens 90%. Eine höhere Abdeckung der Zelle 22 durch die Abdeckung 21 im Herstellzustand ist vorteilhaft, da auf diese Weise die Stromdichte verringert wird.

Die Gesamtheit der Abdeckungen 21 bzw. Verbreiterungen 20 weist vorzugsweise eine Oberfläche auf dem Außenumfang der Gitterstruktur 10 auf, die wenigstens 0,05 mm², insbesondere wenigstens 0,1 mm², insbesondere wenigstens 0,2 mm², insbesondere wenigstens 0,5 mm², insbesondere wenigstens 0,75 mm², insbesondere wenigstens 1 mm², insbesondere wenigstens 2,5 mm², insbesondere wenigstens 1,5 mm², insbesondere wenigstens 1,75 mm², insbesondere wenigstens 2 mm², insbesondere wenigstens 2,5 mm², insbesondere wenigstens 3 mm², beträgt.

Die Abdeckung 21 bzw. die Verbreiterung weist vorzugsweise eine Dicke bzw. Wandstärke auf, die höchstens 20 µm, insbesondere höchstens 15 µm, insbesondere höchstens 10 µm, insbesondere höchstens 8 µm, insbesondere höchstens 6 µm, insbesondere höchstens 5 µm, insbesondere höchstens 4 µm, insbesondere höchstens 3 µm, beträgt. Dadurch werden die Flexibilität und Crimpbarkeit des Systems verbessert.

Die Elektrode, insbesondere die Gitterstruktur 10, ist vorzugsweise derart angepasst, dass sie in ein Zuführsystem, insbesondere einen Katheter, einführbar ist, der einen Innendurchmesser von höchstens 2 mm, insbesondere höchstens 2,8 mm, insbesondere höchstens 1,6 mm, insbesondere höchstens 1,4 mm, insbesondere höchstens 1,2 mm, insbesondere höchstens 1,0 mm, insbesondere höchstens 0,9 mm, insbesondere höchstens 0,8 mm, insbesondere höchstens 0,7 mm, insbesondere höchstens 0,6 mm, insbesondere höchstens 0,52 mm, insbesondere höchstens 0,43 mm, aufweist.

Die Gitterstege 11 weisen, insbesondere im Bereich der mechanisch tragenden Schicht 14, vorzugsweise ein Stegbreite auf, die höchstens 50 µm, insbesondere höchstens 45 µm, insbesondere höchstens 40 µm, insbesondere höchstens 35 µm, insbesondere höchstens 30 µm, insbesondere höchstens 25 µm, insbesondere höchstens 20 µm, insbesondere höchstens 15 µm, beträgt.

Alle Merkmale der Elektrode (Vorrichtung) werden auch im Zusammenhang mit dem Verfahren zur Herstellung der Elektrode offenbart.

Die Erfindung umfasst ferner eine Elektrode für medizinische Anwendungen zur Neuromodulation und/oder Nervenstimulation und/oder neurologischen Signalerfassung, die zur Einfuhr in ein Hohlorgan A eines Körpers komprimierbar und expandierbar ist und mit einer Stromzufuhr gekoppelt oder koppelbar ist. Die Elektrode zeichnet sich durch eine komprimierbare und expandierbare Gitterstruktur 10, die aus Gitterstegen 11 gebildete Zellen 22 aufweist und mit der Stromzufuhr gekoppelt oder koppelbar ist, wobei die Gitterstruktur 10 zumindest bereichsweise wenigstens zwei in radialer Richtung aufeinander angeordnete Schichten 14, 15, 16 aufweist. Der Schichtaufbau der Elektrode wird mithin unabhängig von dem Herstellungsverfahren beansprucht. Alle Merkmale der vorstehend erläuterten Ausführungsbeispiele werden auch im Zusammenhang mit der schichtweise aufgebauten Elektrode gemäß Anspruch 28 offenbart und beansprucht. Dies gilt auch für die Ansprüche 2 bis 27, die ebenfalls im Zusammenhang mit der Elektrode gemäß Anspruch 28 offenbart und beansprucht werden.

### Bezugszeichenliste

- 10: Gitterstruktur
- 11: Gittersteg
- 12: elektrisch leitender Bereich
- 13: elektrisch isolierter Bereich
- 14: mechanisch tragende Schicht
- 15: Isolierschicht
- 16: elektrisch leitende Schicht
- 17: Außenschicht
- 18: Innenschicht
- 19: Zwischenschicht
- 20: Verbreiterung
- 21: Abdeckung
- 22: Zelle
- 23: Versorgungsleitung
- 24: zentrale Versorgungsleitung
- 25: Strukturierung
- 26: Mikrokatheter
- A: Hohlorgan
- B: Feldlinien
- C: orthogonal verlaufende Nervenzellen
- D: parallel verlaufende Nervenzellen
- E: Gegenelektrode

## Patentansprüche

1. Elektrode für medizinische Anwendungen zur Neuromodulation und/oder Nervenstimulation und/oder neurologischen Signalerfassung, die zur Einfuhr in ein Hohlorgan (A) eines Körpers komprimierbar und expandierbar ist und mit einer Stromzufuhr gekoppelt oder koppelbar ist,
**gekennzeichnet durch**
eine komprimierbare und expandierbare freitragende Gitterstruktur (10), die aus Gitterstegen (11) gebildete Zellen (22) aufweist und mit der Stromzufuhr gekoppelt oder koppelbar ist, wobei die freitragende Gitterstruktur (10) **durch** physikalische Gasphasenabscheidung erhalten ist.

2. Elektrode nach Anspruch 1,
**dadurch gekennzeichnet, dass**
das Material der Gitterstruktur (10) eine durch physikalische Gasphasenabscheidung gezielt eingestellte maximale Dichte an Materialinhomogenitäten, wie Gitterbaufehler aufweist.

3. Elektrode nach Anspruch 1 oder 2,
**dadurch gekennzeichnet, dass**
die mittlere Korngröße des Materials der Gitterstruktur (10) weniger als 4 µm, insbesondere weniger als 3 µm, insbesondere weniger als 2 µm, insbesondere weniger als 1,5 µm, insbesondere weniger als 1 µm, insbesondere weniger als 0,5 µm, insbesondere weniger als 250 nm, beträgt.

4. Elektrode nach einem der vorhergehenden Ansprüche,
**dadurch gekennzeichnet, dass**
das Material der Gitterstruktur (10) eine Formgedächtnislegierung oder Stahl oder eine Chrom-Kobalt-Legierung oder ein biologisch abbaubares Material umfasst.

5. Elektrode nach einem der vorhergehenden Ansprüche,
**dadurch gekennzeichnet, dass**
die Gitterstruktur mehrere, insbesondere wenigstens zwei in radialer Richtung aufeinander angeordnete Schichten (14, 15, 16), insbesondere mit unterschiedlichen Funktionen, aufweist.

6. Elektrode nach Anspruch 5,
**dadurch gekennzeichnet, dass**
das die Gitterstruktur (10) wenigstens eine elektrisch leitende Schicht (16) und wenigstens eine mechanisch tragende Schicht (14), insbesondere aus unterschiedlichen Materialien, aufweist.

7. Elektrode nach Anspruch 6,
**dadurch gekennzeichnet, dass**
die elektrisch leitende Schicht (16) außen zur Berührung mit dem Hohlorgan angeordnet und die mechanisch tragende Schicht (14) bezogen auf die elektrisch leitende Schicht (16) radial weiter innen angeordnet ist.

8. Elektrode nach Anspruch 6 oder 7,
**dadurch gekennzeichnet, dass**
die elektrisch leitende Schicht (16) mit einer elektrisch isolierenden Schicht, insbesondere einer Isolierschicht (15), verbunden ist, die auf einer radial innen oder einer radial außen angeordneten Seite der elektrisch leitenden Schicht (16), insbesondere zwischen zwei Schichten angeordnet ist, die mit denselben oder unterschiedlichen elektrischen Spannungen beaufschlagbar sind.

9. Elektrode nach einem der vorhergehenden Ansprüche,
**dadurch gekennzeichnet, dass**
die Gitterstruktur (10) mit wenigstens einem Medikament beladen ist, das durch Anlegen einer elektrischen Spannung freisetzbar ist.

10. Elektrode nach einem der Ansprüche 6 bis 9,
**dadurch gekennzeichnet, dass**
die mechanisch tragende Schicht (14) aus einem biologisch abbaubaren Material gebildet ist.

11. Elektrode nach einem der vorhergehenden Ansprüche,
**dadurch gekennzeichnet, dass**
die Gitterstege (11) eine Außenschicht (17) und eine Innenschicht (18) aufweisen, wobei die Außenschicht (17) eine Verbreiterung (20) umfasst, die sich in Längsrichtung der Gitterstege (11) erstreckt derart, dass die Gitterstege (11) im Bereich der Außenschicht (17) breiter als im Bereich der Innenschicht (18) sind, wobei die Verbreiterung (20) insbesondere wenigstens eine elektrisch leitende Schicht (16) und wenigstens eine mechanisch tragende Schicht (14), insbesondere aus unterschiedlichen Materialien, aufweist, und die elektrisch leitende Schicht (16) die Außenschicht (17) und die mechanisch tragende Schicht (14) eine Zwischenschicht (19) bildet, die zwischen der Außenschicht (17) und der Innenschicht (18) angeordnet ist.

12. Elektrode nach einem der vorhergehenden Ansprüche,
**dadurch gekennzeichnet, dass**
wenigstens ein Gittersteg (11), insbesondere mehrere Gitterstege (11), jeweils mit wenigstens einer Abdeckung (21) verbunden sind, deren Längsrichtung von der Längsrichtung des Gitterstegs (11) abweicht derart, dass sich die Abdeckung (21) in wenigstens eine angrenzende Zelle (22) erstreckt und diese zumindest teilweise abdeckt, insbesondere, dass die Größe und/oder Geometrie der Abdeckungen (21) zumindest teilweise gleich oder unterschiedlich ist und/oder die Anzahl der Abdeckungen (21) auf dem Umfang der Gitterstruktur (10) und/oder in Längsrichtung variiert, insbesondere, dass die Abdeckungen (21) elektrisch leitend durch eine Stromzufuhrschicht verbunden sind, die an der Außenseite oder an der Innenseite der Abdeckungen (21) angeordnet ist und der Anordnung der Gitterstege (11) entspricht, wobei die an der Innenseite der Abdeckungen (21) angeordnete Stromzufuhrschicht die mechanisch tragende Schicht (14) oder eine von der mechanisch tragenden Schicht (14) elektrisch isolierte Schicht bildet.

13. Elektrode nach einem der Ansprüche 7 bis 12,
**dadurch gekennzeichnet, dass**
die außen angeordnete elektrisch leitende Schicht (16), insbesondere die Verbreiterung (20) oder die Abdeckung (21), zur Vergrößerung der Kontaktfläche strukturiert ist.

14. System mit einer Elektrode nach Anspruch 1 sowie einer Stromversorgung, einem Impulsgeber und einer Steuerelektronik, die mit der Elektrode gekoppelt oder koppelbar sind, insbesondere wobei
wenigstens eine weitere Elektrode nach Anspruch 1 und/oder wenigstens eine weitere an sich bekannte Elektrode zur medizinischen Anwendung, wobei die Elektrode mit der weiteren Elektrode wirkverbunden oder wirkverbindbar ist derart, dass eine bipolare Stromführung einstellbar ist.

15. Verfahren zur Herstellung einer Elektrode nach Anspruch 1, bei dem die Gitterstruktur durch physikalische Gasphasenabscheidung hergestellt wird, wobei ein Material auf einem Substrat schichtweise abgeschieden und strukturiert wird derart, dass eine freitragende Gitterstruktur (10) gebildet wird.

## Claims

1. An electrode for medical applications for neuromodulation and/or nerve stimulation and/or neurological signal detection, which is able to be compressed and expanded for introduction into a hollow organ (A) of a body and is coupled or able to be coupled to a current supply,
**characterized by**
a compressible and expandable self-supporting lattice structure (10), which has cells (22) formed from lattice webs (11) and is coupled or able to be coupled to the current supply, wherein the self-supporting lattice structure (10) is obtained by physical vapour deposition.

2. The electrode according to claim 1,
**characterized in that**
the material of the lattice structure (10) has a maximum density of material inhomogeneities, such as lattice structure defects, adjusted in a targeted manner by physical vapour deposition.

3. The electrode according to claim 1 or 2,
**characterized in that**
the average grain size of the material of the lattice structure (10) is less than 4 µm, in particular less than 3 µm, in particular less than 2 µm, in particular less than 1.5 µm, in particular less than 1 µm, in particular less than 0.5 µm, in particular less than 250 nm.

4. The electrode according to one of the preceding claims,
**characterized in that**
the material of the lattice structure (10) comprises a shape memory alloy or steel or a chromium/cobalt alloy or a biologically degradable material.

5. The electrode according to one of the preceding claims,
**characterized in that**
the lattice structure has several, in particular at least two layers (14, 15, 16) arranged in radial direction on one another, in particular having different functions.

6. The electrode according to claim 5,
**characterized in that**
the lattice structure (10) has at least one electrically conductive layer (16) and at least one mechanically supporting layer (14), in particular of different materials.

7. The electrode according to claim 6,
**characterized in that**
the electrically conductive layer (16) is arranged externally for contact with the hollow organ and the mechanically supporting layer (14) is arranged radially further inward in relation to the electrically conductive layer (16).

8. The electrode according to claim 6 or 7,
**characterized in that**
the electrically conductive layer (16) is connected to an electrically insulating layer, in particular an insulator layer (15), which is arranged on a side of the electrically conductive layer (16) arranged radially inward or radially outward, in particular is arranged between two layers to which the same or different electrical voltages are able to be applied.

9. The electrode according to one of the preceding claims,
**characterized in that**
the lattice structure (10) is loaded with at least one medicament, which is able to be released by application of an electrical voltage.

10. The electrode according to one of claims 6 to 9,
**characterized in that**
the mechanically supporting (14) layer is formed from a biologically degradable material.

11. The electrode according to one of the preceding claims,
**characterized in that**
the lattice webs (11) have an outer layer (17) and an inner layer (18), wherein the outer layer (17) comprises a widening (20), which extends in the longitudinal direction of the lattice webs (11) such that the lattice webs (11) are wider in the region of the outer layer (17) than in the region of the inner layer (18), wherein the widening (20) has at least one electrically conductive layer (16) and at least one mechanically supporting layer (14), in particular of different materials, and the electrically conducting layer (16) forms the outer layer (17) and the mechanically supporting layer (14) forms an intermediate layer (19), which is arranged between the outer layer (17) and the inner layer (18).

12. The electrode according to one of the preceding claims,
**characterized in that**
at least one lattice web (11), in particular several lattice webs (11), are respectively connected to at least one cover (21), the longitudinal direction of which differs from the longitudinal direction of the lattice web (11) such that the cover (21) extends into at least one adjacent cell (22) and at least partially covers the latter, in particular that the size and/or geometry (21) of the covers (21) is at least partially identical or different and/or the number of covers (21) varies on the circumference of the lattice structure (10) and/or in longitudinal direction, in particular that the covers (21) are connected in an electrically conductive manner by an electrical supply layer, which is arranged on the outer side or on the inner side of the covers (21) and corresponds to the arrangement of the lattice webs (11), wherein the electrical supply layer arranged on the inner side of the covers (21) forms the mechanically supporting layer (14) or a layer electrically insulated from the mechanically supporting layer (14).

13. The electrode according to one of claims 7 to 12,
**characterized in that**
the externally arranged electrically conductive layer (16), in particular the widening (20) or the cover (21) is structured in order to increase the contact area.

14. A system having an electrode according to claim 1 and a current supply, a pulse generator and control electronics, which are coupled or able to be coupled to the electrode, in particular wherein
at least one further electrode according to claim 1 and/or at least one further electrode known per se for medical application,
wherein the electrode is operatively connected or able to be operatively connected to the further electrode, such that a bipolar current supply is able to be set up.

15. A method for producing an electrode according to claim 1, in which the lattice structure is produced by physical vapour deposition, wherein a material is deposited in layers on a substrate and structured such that a self-supporting lattice structure (10) is formed.

## Revendications

1. Electrode pour utilisations médicales pour la neuromodulation et/ou neurostimulation et/ou la détection de signal neurologique, laquelle peut se comprimer et s'expanser pour l'introduction dans un organe creux (A) d'un corps et laquelle est couplée, ou peut être couplée à une alimentation électrique,
**caractérisée par**
une structure en treillis (10) autoporteuse comprimable et expansible qui présente des cellules (22) formées par des barres de treillis (11) et qui est couplée, ou peut être couplée à l'alimentation électrique, dans laquelle la structure en treillis autoporteuse (10) est obtenue par dépôt physique en phase vapeur.

2. Electrode selon la revendication 1,
**caractérisée en ce que** le matériau de la structure en treillis (10) présente une densité maximale, réglée de façon ciblée par dépôt physique en phase vapeur, d'inhomogénéités de matériau, telles des défauts de construction de treillis.

3. Electrode selon la revendication 1 ou 2,
**caractérisée en ce que** la granulométrie moyenne du matériau de la structure en treillis (10) est de moins de 4 µm, en particulier de moins de 3 µm, en particulier de moins de 2 µm, en particulier de moins de 1,5 µm, en particulier de moins de 1 µm, en particulier de moins de 0,5 µm, en particulier de moins de 250 nm.

4. Electrode selon l'une des revendications précédentes,
**caractérisée en ce que** le matériau de la structure en treillis (10) comprend un alliage à mémoire de forme ou acier ou un alliage de chrome-cobalt ou un matériau biodégradable.

5. Electrode selon l'une des revendications précédentes,
**caractérisée en ce que** la structure en treillis présente plusieurs, en particulier au moins deux couches (14, 15, 16) disposées de façon superposée en direction radiale, en particulier avec des fonctions différentes.

6. Electrode selon la revendication 5,
**caractérisée en ce que** la structure en treillis (10) présente au moins une couche électroconductrice (16) et au moins une couche de support mécanique (14), en particulier en matériaux différents.

7. Electrode selon la revendication 6,
**caractérisée en ce que** la couche électroconductrice (16) est disposée à l'extérieur pour le contact avec l'organe creux et **en ce que** la couche de support mécanique (14) est disposée radialement plus à l'intérieur compte tenu de la couche électroconductrice (16).

8. Electrode selon la revendication 6 ou 7,
**caractérisée en ce que** la couche électroconductrice (16) est reliée à une couche électriquement isolante, en particulier une couche d'isolation (15), laquelle est disposée sur un côté disposé radialement à l'intérieur ou radialement à l'extérieur de la couche électroconductrice (16), en particulier entre deux couches, auxquelles on peut appliquer les mêmes tensions électriques ou des tensions électriques différentes.

9. Electrode selon l'une des revendications précédentes,
**caractérisée en ce que** la structure en treillis (10) est chargée avec au moins un médicament pouvant être libéré par application d'une tension électrique.

10. Electrode selon l'une des revendications 6 à 9,
**caractérisée en ce que** la couche de support mécanique (14) est formée par un matériau biodégradable.

11. Electrode selon l'une des revendications précédentes,
**caractérisée en ce que** les barres de treillis (11) présentent une couche extérieure (17) et une couche intérieure (18), dans laquelle la couche extérieure (17) comprend un élargissement (20) s'étendant en direction longitudinale des barres de treillis (11) de manière à ce que les barres de treillis (11) soient plus larges dans la zone de la couche extérieure (17) que dans la zone de la couche intérieure (18), dans laquelle l'élargissement (20) présente en particulier au moins une couche électroconductrice (16) et au moins une couche de support mécanique (14), en particulier en matériaux différents, et la couche électroconductrice (16) formant la couche extérieure (17) et la couche de support mécanique (14) formant une couche intermédiaire (19) qui est disposée entre la couche extérieure (17) et la couche intérieure (18).

12. Electrode selon l'une des revendications précédentes,
**caractérisée en ce qu'**au moins une barre de treillis (11), en particulier plusieurs barres de treillis (11), sont respectivement reliées à au moins un recouvrement (21) dont la direction longitudinale diffère de la direction longitudinale de la barre de treillis (11) de manière à ce que le recouvrement (21) s'étend dans au moins une cellule (22) adjacente et recouvre au moins partiellement celle-ci, en particulier **en ce que** la grandeur et/ou géométrie des recouvrements (21) est au moins partiellement égale ou différente et/ou **en ce que** le nombre de recouvrements (21) varie sur le pourtour de structure en treillis (10) et/ou en direction longitudinale, en particulier en ce que les recouvrements (21) sont reliés de manière électroconductrice par une couche d'alimentation électrique qui est disposée sur le côté extérieur ou sur le côté intérieur des recouvrements (21) et qui correspond à l'agencement des barres de treillis (11), dans laquelle la couche d'alimentation électrique disposée sur le côté intérieur des recouvrements (21) forme la couche de support mécanique (14) ou une couche isolée électriquement par rapport à la couche de support mécanique (14).

13. Electrode selon l'une des revendications 7 à 12,
**caractérisée en ce que** la couche électroconductrice (16) disposée à l'extérieur, en particulier l'élargissement (20) ou le recouvrement (21), est structuré(e) pour l'agrandissement de la surface de contact.

14. Système avec une électrode selon la revendication 1 ainsi qu'avec une alimentation électrique, un générateur d'impulsions et une électronique de commande couplés, ou pouvant être couplés à l'électrode, dans lequel en particulier
au moins une autre électrode selon la revendication 1 et/ou au moins une autre électrode connue en tant que telle pour l'utilisation médicale,
dans lequel l'électrode est reliée de manière fonctionnelle, ou peut être reliée de manière fonctionnelle à l'autre électrode de façon à ce qu'une conduction de courant bipolaire puisse être réglée.

15. Procédé pour la fabrication d'une électrode selon la revendication 1, dans lequel la structure en treillis est produite par dépôt physique en phase vapeur, dans lequel un matériau est déposé par couches sur un substrat et est structuré de manière à former une structure en treillis (10) autoporteuse.
